# EUROPEAN PATENT SPECIFICATION

(11) **EP 2 162 016 B1**
(45) Date of publication and mention of the grant of the patent: **11.04.2012**
(21) Application number: 07766613.9
(22) Date of filing: 06.07.2007
(51) Int. Cl.: A23L 1/00, A23G 4/20, A23G 4/08, A23G 4/06, A61K 9/00

(54) **CHEWING GUM GRANULES FOR COMPRESSED CHEWING GUM**
KAUGUMMIGRANULAT FÜR KOMPRIMIERTEN KAUGUMMI
GRANULÉS DE GOMME À MÂCHER POUR GOMME À MÂCHER COMPRIMÉE

(43) Date of publication of application: 17.03.2010
(73) Proprietor: Gumlink A/S, 7100 Vejle (DK)
(72) Inventor: ANDERSEN, Carsten, 7100 Vejle (DK); TOPSØE, Martin, 7100 Vejle (DK); THORENGAARD, Bitten, 7120 Vejle Øst (DK)
(74) Representative: Mikkelsen, Hans Kristian Saaby
(86) International application number: PCT/IB2007/001902
(87) International publication number: WO 2009/007770

(56) References cited:
- EP-A- 0 151 344
- EP-A- 1 427 292
- WO-A-02/051391
- WO-A-2004/004479
- WO-A-2004/032644
- WO-A-2004/040995
- WO-A-2007/022317

## Description

### FIELD OF THE INVENTION

The invention relates to the field of chewing gum and in particular to compressed chewing gum.

### TECHNICAL BACKGROUND AND PRIOR ART

Compressed chewing gum has been known for decades and a number of prior art documents have indicated that compressed chewing gum has the advantage of being especially suitable for delivery of active ingredients. As some active ingredients tend to become unstable when exposed to heat or certain other conditions associated to the manufacture of conventional chewing gum, compressed chewing gum has often been preferred as delivery vehicle.

While the prior art has disclosed some possible ways to produce compressed chewing gum, the most prominent method is to produce compressed chewing gum by particulation of conventional chewing gum, producing irregular small granules, often referred to as chewing gum powder, which is then compressed to a coherent compressed chewing gum.

EP 1 427 292 discloses a method for the production of chewing gum powder, which is then subsequently compressed into a final chewing gum tablet. The powder is obtained through cooling and grinding of a soft basic gum and the result is irregular powder granules. The irregular shaping results in a very large surface area of the powder granules where each surface subdivision holds the potential for sticking. While addition of anti-agglutination agent has been suggested, the amount would not be sufficient to cover the surface of the powder granules. Mechanical locking caused by the shape of the powder granules combined with an insufficient amount of anti-agglutination agent will inferior the flow properties of a composition of chewing gum powder.

EP 0 151 344 discloses chewing compositions comprising a mixt. of: (a) granules comprising chewing gum base, grinding aid and sweetener; and (b) a compression aid comprising at least 2 of a lubricant, a glidant and an anti-adherent; where the moisture content is 2-8 wt.% of the gum composition. The granules are made by the use of a milling process, which inevitably will not result in substantially ball-shaped granules.

WO 2005/011397 discloses a chewing gum base comprising high molecular weight polyisobutylene and which is free of non-silica filler. The gum base may be extruded or cast into any desirable shape.

### SUMMARY OF THE INVENTION

The invention relates to a chewing gum granule containing gum base, said chewing gum granule being substantially ball-shaped, said chewing gum granule having an average diameter below 1900 µm, and the surface of said chewing gum granule being provided with 0.5 to 18% by weight of free-flowing agent.

According to the invention, it has been established that the tabletting process for a compressed chewing gum delivers an improved product with regard to i.e. stability and texture when the amount of free-flowing agent provided on the surface of the chewing gum granule is controlled according to the invention in order to avoid a too high level of free-flowing agent in the compressed chewing gum and thereby experience disintegration of compressed chewing gum comprising chewing gum granules according to the invention.

However, according to the invention, it has also been realized that the complete absence of free-flowing agent on the surface of the chewing gum granules results in a more difficult handling during e.g. transportation and storage as the chewing gum granules without free-flowing agent will tend to stick together and thereby result in an increased necessary effort when the chewing gum granules are to be separated for use in a compressed chewing gum. The stickiness may even turn so bad that consequently large quantities of chewing gum granules must be dumped.

With the present invention it has surprisingly been obtained that a favorable amount of free-flowing agent within an interval of 0.5 to 18% by weight provided on the surface of said chewing gum granules is capable of ensuring an improved stability and texture of the compressed chewing gum and at the same time ensure that the chewing gum granules during transportation and storage have improved flow-properties and do not stick to be inseparable.

During transportation of granules, e.g. through pipelines in a production process, it is not only very advantageous that the granules do not stick to each other but at the same time do not mechanically lock each other. With good flow-properties the granules may be lead to flow in a desired direction without the same need for active maintenance as could be the case for mechanically locked granules.

It has been found that for granules with free-flowing agent below about 0.5% by weight problems may arise with sticking between the granules. Small amounts of free-flowing agent causes that every spot on the surface of each granule is not covered, whereby the risk is higher of two badly covered spots touching each other whereby two granules may stick together and thereby worsen the flow abilities of the composition.

It has been found that for granules with free-flowing agent above about 18% by weight problems may arise when compressing the granules because the high amount of free-flowing agent weakens the effective packing of granules in a compressed chewing gum.

Furthermore, ball-shaped granules according to the invention may undergo an improved dense packaging when compressed as compared with cooled and grinded granules as the result of compression of ball-shaped granules will result in more regular building elements. Moreover, chewing gum granules with sizes of an average diameter below 1900 µm may be easier to pack densely during compression of the granules as these naturally pack denser prior to compression than larger granule sizes.

Moreover, it has been obtained that the use of ball-shaped granules results in improved flowing properties of the granules besides providing a smaller surface/volume ratio for the granule resulting in a lesser need for high amounts of free-flowing agent.

Typically in prior art particles obtained through cooling and grinding are used, through which process irregular particles with large surface areas compared to volume is obtained. A problem with these prior art particles is that there is a tendency towards applying large amounts of anti-agglutination agent in order to cover the whole surface area. However, the amount can not be raised as desired for anti-sticking purposes, as the texture of the final produced chewing gum will be inferior. Thereby the product may become crumbling or easily disintegrate. With the present invention this problem is overcome.

Typically, prior art does not deal with free-flow properties even though acceptable texture and anti-sticking of granules may at least partly be dealt with.

With the present invention the ball-shaped shape of the granules according to embodiments of the invention results in that mechanical locking in a chewing gum composition may be minimized and further that a lower need for high amounts of free-flowing agent in order to obtain satisfying results may occur.

In an embodiment of the invention, said chewing gum granule has an average diameter below 1600 µm.

In an embodiment of the invention, said chewing gum granule has an average diameter below 1400 µm.

In an embodiment of the invention, said chewing gum granule has an average diameter below 1200 µm.

In an embodiment of the invention, said chewing gum granule has an average diameter above 200 µm.

In an embodiment of the invention, said chewing gum granule has an average diameter above 400 µm.

In an embodiment of the invention, the surface of said chewing gum granule is provided with more than 1% by weight of free-flowing agent.

In an embodiment of the invention, the surface of said chewing gum granule is provided with more than 2% by weight of free-flowing agent.

In an embodiment of the invention the surface of said chewing gum granule is provided with less than 16% by weight of free-flowing agent.

In an embodiment of the invention, the surface of said granule is provided with less than 14% by weight of free-flowing agent.

In an embodiment of the invention, the surface of said chewing gum granule is provided with less than 12% by weight of free-flowing agent.

In an embodiment of the invention, the surface of said chewing gum granule is provided with less than 10% by weight of free-flowing agent.

In an embodiment of the invention, the surface of said granule is provided with 4 to 8% by weight of free-flowing agent.

According to an embodiment of the invention, the amount of free-flowing agent is matched to the average diameter of the chewing gum granule as an increasing average diameter of a granule results in an increasing surface area of the granule and thereby more free-flowing agent is needed to obtain the same result. E.g. for a chewing gum granule with an average diameter of approximately 1200 µm, between 4 and 8% by weight of free-flowing agent is advantageous.

In an embodiment of the invention, the surface of said granule is smooth, said granule being substantially without protrusions, depressions or fissures.

According to an embodiment of the invention, the granule is shaped in order to provide a favorable ratio between volume and surface area, which is achieved for a surface as smooth as possible. In this way the amount of free-flowing agent needed to obtain satisfying non-sticking properties is lower and the risk of experiencing difficulties due to too much free-flowing agent when tabletting is reduced.

In an embodiment of the invention, the smooth surface is obtained by means of expansion.

According to an embodiment of the invention, the granule is produced in an extruder inside of which the pressure is markedly higher than outside the extruder. This causes the granules to undergo an expansion after being cut into pieces when leaving the extruder, which results in the surface being smooth.

In an embodiment of the invention, the smooth surface is obtained by means of expansion in a liquid media.

In an embodiment of the invention, the smooth surface is obtained by means of expansion in air.

In an embodiment of the invention, said free-flowing agent has a particle size of less than 80 µm.

Preferably the free-flowing agent is particular with a particle size less than the average diameter of the chewing gum granules in order to efficiently be able to distribute on the surface of the chewing gum granules.

The word particle throughout this document should be understood as broadly as possibly being powder, granules, agglomerates or the like. In the same manner the word granule may be understood as a particle and generally the four terms may be used interchangeably.

In an embodiment of the invention, said free-flowing agent has a particle size of less than 70 µm.

In an embodiment of the invention, said free-flowing agent has a particle size of less than 60 µm.

In an embodiment of the invention said free-flowing agent has a particle size of less than 50 µm.

In an embodiment of the invention, said free-flowing agent has a particle size above 2 µm.

In an embodiment of the invention, said free-flowing agent has a particle size between 5 and 40µm.

In an embodiment of the invention, said free-flowing agent is attached to said chewing gum granule by mechanical mixing.

The term "attached to" throughout this document should be understood as synonymous to "deposited on", "adhered unto" and the like.

In an embodiment of the invention, said free-flowing agent is attached to said chewing gum granule by dusting.

In an embodiment of the invention, said free-flowing agent is attached to said chewing gum granule by spraying.

The free-flowing agent may be attached to the chewing gum granules in different ways, such as by mechanical mixing, dusting or spraying. No matter which of these processes is being used, the free-flowing agent is advantageously attached to the granules at a stage after expansion to avoid sticking between the granules.

In an embodiment of the invention, said free-flowing agent is attached to said chewing gum granule by introducing an electrostatic charge to said free-flowing agent.

In an embodiment of the invention, the free-flowing agent is provided on the surface of the chewing gum granule by electrostatic means. An electrostatic charge is introduced into the free-flowing agent prior to application by delivering a voltage from a voltage source to electrode of from about 10,000 to about 100,000 volts. Thereby the free-flowing agent can be applied as forming a coating on the surface of the chewing gum granules, and means for pulling off excess free-flowing agent may be provided if desired. In this way it is possible to deposit a suitable amount of free-flowing agent on the surface of the chewing gum granules to avoid sticking. An example of electrostatical application of a powder can be seen in e.g. WO 2006/073598.

In an embodiment of the invention, said chewing gum granule comprises active ingredients.

The chewing gum granule may comprise one or more of the active ingredients chosen from the lists of active ingredients mentioned in this document.

In an embodiment of the invention, said chewing gum granule substantially consists of gum base.

When a chewing gum granule as mentioned herein substantially consists of gum base, the result is gum base granules typically based mainly on natural and/or synthetic resins and/or elastomers. Such gum base granules may find application in combination with a variety of flavoring, sweetening and so on.

In an embodiment of the invention, said chewing gum granule comprises chewing gum ingredients.

In an embodiment of the invention, said chewing gum ingredients comprises softener, flavor, sweetener and optionally filler.

In embodiments of the invention, the chewing gum granule may comprise chewing gum ingredients such as filler, coloring agent, flavoring agent, high-intensity sweetener, bulk sweetener, softener, emulsifier, acidulant, antioxidant, further conventional chewing gum ingredients and more.

In an embodiment of the invention, said free-flowing agent has a particle size of less than 1/10 of the average diameter of said chewing gum granules.

In case the particle size of the free-flowing agent is comparable to or just more than about 1/5 of the average diameter of the chewing gum granules it will be difficult to cover the surface of the granules with the free-flowing agent particles without ending up with too high amounts of free-flowing agent on the granules in order to obtain a satisfying result in the tabletting process.

Moreover, it has been discovered by the applicant that smaller particle sizes of the free-flowing agent facilitates very advantageous properties when handling the granules.

To lower the particle size of the free-flowing agent is one way in many whereby an improvement of free-flow properties may be seen. Typically the friction of the granules will be lowered by a layer of free-flowing agent, whereby the free-flow will be improved. It is, however, not desirable that the surfaces of the granules are completely frictionless.

A smaller particle size of the free-flowing agent will typically cause a thinner layer of free-flowing agent to be necessary in order to cover the complete surface of the granule; however, for particle sizes too low other problems may arise with contact between free-flowing agents.

In an embodiment of the invention, said free-flowing agent is selected from the group consisting of talc, CaCO₃, Mg-stearate, SiO₂, gum Arabic, starch such as corn starch, Na-CMC, methylcellulose, saccharide, polyols, fine-powdered bulk sweetener such as xylitol, isomalt or maltitol, active ingredients, any other suitable agent for improving the flowing properties, or any combination thereof.

In another embodiment of the invention, said free-flowing agent is selected from the group consisting of calcium stearate, zinc stearate, glycerine mono-stearate, calcium silicate cellulose, magnesium silicate and magnesium tri-silicate,

A free-flowing agent according to the present invention is used to minimize agglutination of the granules and to increase flowing abilities of the granules. The surface of the granules without free-flowing agent may be sticky and the addition of free-flowing agent may thus aid in avoiding sticking of the granules. Moreover, a layer of free-flowing agent on the surface of the granules may improve the friction properties between the granules to in order to ensure an improved free-flow in a composition of granules.

Suitable agents to be used as free-flowing agents may be any of the above-mentioned or any other agent, which is capable of minimizing sticking and to ensure a surface of the granules which will improve the free-flow of the composition.

In an embodiment of the invention, said free-flowing agent is provided as a film-coat.

The free-flowing agent provided on the surface may in an embodiment of the invention be the same as filler applied in the chewing gum granules. The total amount of free-flowing agent in the chewing gum may therefore in total be higher than the amount provided on the surface.

In an embodiment of the invention said, chewing gum granule is substantially free of free-flowing agent.

According to an embodiment of the invention, the amount of free-flowing agent provided on the surface of the granule is the total amount of free-flowing agent of the granule.

Moreover the invention relates to a chewing gum composition comprising chewing gum granules according to any of the claims 1-19.

In an embodiment of the invention, at least 95% by weight of said chewing gum granules have an average diameter of less than 1900 µm.

In a composition of several chewing gum granules the sizes of the granules will not be exactly the same, and some granules being larger and some being smaller than the majority of the granules do not degrade the usefulness of the composition; however, preferably at least 95% by weight of said chewing gum granules have an average diameter of less than 1900 µm.

In an embodiment of the invention, at least 80% by weight of said chewing gum granules have an average diameter of less than 1500 µm.

In an embodiment of the invention, said chewing gum granules are substantially homogeneous in shape.

The flowing properties of a composition of chewing gum granules as described herein may be improved when said granules are substantially homogenous in shape with a ball-shaped appearance such as herein described.

In an embodiment of the invention, more than 95% of said chewing gum granules have an average diameter deviating less than 30% from the mean value diameter of said chewing gum granules.

It has been discovered that a composition wherein less than 95% by weight of the granules deviates less than 30% from the mean value diameter of said chewing gum granules show improved properties with regard to the tabletting process.

Moreover, when the majority of the granules deviates less than 30% from the mean value diameter of said chewing gum granules the problem of segregation of different granules will be detectably less.

According to an embodiment of the invention, more than 95% of said chewing gum granules have an average diameter deviating less than 20% from the mean value diameter of said chewing gum granules.

In an embodiment of the invention, more than 95% of said chewing gum granules have an average diameter deviating less than 10% from the mean value diameter of said chewing gum granules.

According to an embodiment of the invention, more than 95% of said chewing gum granules have an average diameter deviating less than 5% from the mean value diameter of said chewing gum granules.

In an embodiment of the invention, more than 95% of said chewing gum granules substantially have the same average diameter.

In an embodiment of the invention, more than 95% of said chewing gum granules substantially have the same density.

In an embodiment of the invention, more than 95% of said chewing gum granules substantially have the same surface characteristics.

Surface characteristics in this context refer to smoothness of the surface, variation in the surface profile, etc. A higher degree of uniformity between the granules aids to avoid granule segregation. Moreover, the quality of the composition is improved and the handling of the composition is eased.

In an embodiment of the invention, at least 40% of said chewing gum granules are substantially ball-shaped.

The ball-shaped granule will have a markedly lower surface area/granule volume ratio than other granule shapes such as e.g. flakes or star-shaped granules. Thereby the ball-shaped granule works as a means for lowering the amount of needed free-flowing agent for each granule. The effective surface area is optimized and with this characteristics the granules will be free-flowing and may be handed in a mechanically ideal way.

As a matter of course ball-shaped items are better at rolling, and hence the term "ball-shaped" in this document is meaning ball-shaped enough to provide good flowing abilities.

In an embodiment of the invention, at least 70% of said chewing gum granules are substantially ball-shaped.

In an embodiment of the invention, at least 95% of said chewing gum granules are substantially ball-shaped.

According to an embodiment of the invention, tablet material is present in the chewing gum composition. The amount of tablet material in the composition may vary from substantially 0% by weight up to substantially 100% by weight.
Tablet material in this document may be combinations of e.g. filler, coloring agent, flavoring agent, high-intensity sweetener, bulk sweetener, softener, emulsifier, acidulant, antioxidant and more.
Tablet material may constitute separate granules, be part of the granules, mixed with the granules in compositions or even be independent modules.

In an embodiment of the invention, said tablet material comprises bulk sweetener in an amount of 20-100% by weight, preferably 40-100% by weight.

In an embodiment of the invention, said tablet material comprises flavor in an amount of 0-60% by weight.

In an embodiment of the invention said tablet material comprises polyols.

In an embodiment of the invention, the ratio between chewing gum granules and tablet material is between 1:5 and 100:1.

In an embodiment of the invention, said tablet material is compressible.

In an embodiment of the invention, said tablet material is agglomerated.

In an embodiment of the invention, said tablet material comprises active ingredients.

In an embodiment of the invention, said tablet material has a mean value diameter between 100 and 1600 µm.

In an embodiment of the invention, 95% by weight of said tablet material and said chewing gum granules have a mean value diameter of between 100 and 600 µm.

According to an embodiment of the invention, the mean value diameter of said tablet granules and said chewing gum granules is between 100 and 400 µm.

In an embodiment of the invention, 95% by weight of said tablet material and said chewing gum granules have a mean value diameter of between 600 and 1600 µm.

According to an embodiment of the invention, the mean value diameter of said tablet granules and said chewing gum granules is between 1000 and 1600 µm.

In an embodiment of the invention, more than 80% by weight of said tablet material and said chewing gum granules have an average diameter deviating less than 30% from the mean value diameter of said tablet material and said granules.

When the majority of the tablet material and the chewing gum granules deviates less than 30% from the mean value diameter of said tablet material and said chewing gum granules the problem of segregation in the mixture will be detectably smaller.

In an embodiment of the invention, more than 95% by weight of said tablet material and said chewing gum granules have an average diameter deviating less than 30% from the mean value diameter of said tablet material and said granules.

In an embodiment of the invention, the mean value diameter of said tablet material and the mean value diameter of said chewing gum granules are comparable.

Preferably the mean value diameter of the tablet material and the chewing gum granules are substantially identical, whereby segregation is minimized and a more uniform distribution between tablet material and chewing gum granules is obtained. Moreover, the invention relates to a compressed module obtained by compressing the composition.

According to an embodiment of the invention, a compressed module is obtained by compressing a composition as herein described. A single module may in itself constitute a piece of chewing gum or a number of modules may be compressed on top of each other to provide a multi-layer chewing gum.

Moreover, the invention relates to a compressed chewing gum comprising at least one compressed module.

In an embodiment of the invention, the compressed chewing gum according to claim 44 comprises at least two individual coherent compressed modules.

In an embodiment of the invention, the compressed chewing gum according to claim 44 or 45 comprises at least three individual coherent compressed modules.

According to an embodiment of the invention, the compressed chewing gum is constructed by two individual coherent compressed modules.

According to an embodiment of the invention, the compressed chewing gum is constructed by three individual coherent compressed modules.

In an embodiment of the invention, the compressed chewing gum comprises at least four individual coherent compressed modules.

In an embodiment of the invention, at least one of said modules is a compressed chewing gum module comprising a composition according to any of the claims 1-14.

In an embodiment of the invention, at least one of said modules is a compressed tablet module comprising a composition of tablet material being substantially free of gum base material.

According to an embodiment of the invention, a compressed chewing gum is manufactured comprising at least two modules of which at least one module consists of tablet material and is substantially free of gum base material such as resin and elastomer.

In an embodiment of the invention, at least one of said modules is a compressed chewing gum module and at least one other compressed module is a compressed tablet module comprising a composition of tablet material substantially free of gum base material.

In an embodiment of the invention, at least one of said compressed modules comprises one or more active ingredients.

In an embodiment of the invention, at least one of said compressed tablet modules comprises one or more active ingredients.

In an embodiment of the invention, said one or more active ingredients are agglomerated with gum base to obtain granules having an average diameter between 200 µm and 2000 µm.

In an embodiment of the invention, said one or more active ingredients are agglomerated with gum base to obtain granules having an average diameter above 400 µm.

In an embodiment of the invention, said one or more active ingredients are agglomerated with chewing gum to obtain granules having an average diameter between 200 µm and 2000 µm.

In an embodiment of the invention, said one or more active ingredients are agglomerated with chewing gum to obtain granules having an average diameter above 400 µm.

In an embodiment of the invention, said one or more active ingredients are agglomerated with tablet material to obtain granules having an average diameter between 200 µm and 2000 µm.

In an embodiment of the invention, one or more of said tablet granules are an active ingredient-comprising tablet granule.

In an embodiment of the invention, said granules are mixed in an appropriate mixing ratio and subsequently compressed.

In an embodiment of the invention, said chewing gum granules are mixed with chewing gum powder obtained through the following steps:
a) cooling of a gum base to a temperature of between about 0 and -273°C,
b) grinding of the cooled gum base,
c) optional mixing of the powder thus obtained with at least one free-flowing agent, to obtain a chewing gum blend.

Even though the stated arguments concerning the favorable results obtained by using ball-shaped granules still persists it is possible to mix these granules with a non-ball-shaped chewing gum powder which is the result of a quite different process. The amount of chewing gum powder should be kept low as compared to the chewing gum granules to avoid loosing the favorable abilities mentioned previously.

In an embodiment of the invention, said chewing gum granules are mixed with chewing gum powder obtained through the following steps:
a) mixing of a gum base with at least one sweetener and, optionally, at least one other typical chewing-gum ingredient,
b) cooling of the mixture thus obtained to a temperature of between about 0 and -273 °C,
c) grinding of the cooled gum base,
d) mixing of the powder thus obtained with at least one free-flowing agent,
e) optional mixing of the powder thus obtained with one or more of sweeteners, flavorings, colorings, food acids or other active ingredients,
to obtain a chewing gum blend.

In an embodiment of the invention, said chewing gum blend is compressed.

In an embodiment of the invention, one or more of said compressed chewing gum blends are used as a module in a compressed multi-layer chewing gum.

In an embodiment of the invention, said active ingredient is selected from the group consisting of pharmaceuticals, nutraceuticals, medicaments, nutrients, nutritional supplements, drugs, dental care agents, herbals, and the like and combinations thereof.

In an embodiment of the invention, said active ingredient is selected from the ATC anatomical groups consisting of agents acting on:

**A** alimentary tract and metabolism, **B** blood and blood forming organs, **C** cardiovascular system, **D** dermatologicals, **G** genito urinary system and sex hormones, **H** systemic hormonal preparations, **J** antiinfectives for systemic use, **L** antineoplastic and immunomodulating agents, **M** musculo-skeletal system, **N** nervous system, **P** antiparasitic products, insecticides and repellents, **R** respiratory system and S sensory organs, **V** various, or any combination thereof.

In an embodiment of the invention, said active ingredient is selected from the ATC therapeutical groups consisting of:
**A01** Stomatological preparations, **A02** Drugs for acid related disorders, **A04** Antiemetics and antinauseants, **A06** Laxatives, **A07** Antidiarrheals, intestinal anti-inflammatory/anti-infective agents, **A08** Antiobesity preparations, excluding diet products, **A10** Drugs used in diabetes, **A11** Vitamins, **A12** Mineral supplements, **B01** Antithrombotic agents, **B03** Antianemic preparations, **C01** Cardiac therapy, **C10** Serum lipid reducing agents, **D01** Antifungals for dermatological use, **G03** Sex hormones, **G04** Urologicals, **M01** Anti-inflammatory and antirheumatic products, **M09** Other drugs for disorders of the musculo-skeletal system, **N01** Anesthetics, **N02** analgesics, **N07** Other nervous system drugs, **R01** Nasal preparations, **R02** Throat preparations, **R03** Drugs for obstructive airway diseases, **R05** Cough and cold preparations, and **R06** Antihistamines for systemic use, **V01** allergens, **V04** diagnostic agents, or any combination thereof.

In an embodiment of the invention, said active ingredient is selected from the therapeutical groups consisting of:
Antipyretic, Anti allergic, Anti-arrytmic, Appetite suppressant, Anti-inflammatory, Broncho dilator, Cardiovascular drugs, Coronary dilator, Cerebral dilator, Peripheral vasodilator, Anti-infective, Psychotropic, Anti-manic, Stimulant, Decongestant, Gastro-intestinal sedative, Sexual dysfunction agent, Desinfectants, Anti-anginal substance, Vasodilator, Anti-hypertensive agent, Vasoconstrictor, Migraine treating agent, Anti-biotic, Tranquilizer, Anti-psychotic, Anti-tumor drug, Anticoagulant, Hypnotic, Sedative, Anti-emetic, Anti-nauseant, Anti-convulsant, Neuromuscular agent, Hyper and hypoglycaemic, Thyroid and anti-thyroid, Diuretic, Antispasmodic, Uterine relaxant, Anoretic, Spasmolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretic, Anti-flatulent, Betablocker, Teeth Whitener, Enzyme, Co-enzyme, Protein, Energy Booster, Fiber, Probiotics, Prebiotics, Antimicrobial agent, NSAID, Anti-tussives, Decongestrants, Anti-histamines, Anti-diarrheals, Hydrogen antagonists, Proton pump inhibitors, General nonselective CNS depressants, General nonselective CNS stimulants, Selectively CNS function modifying drugs, Antiparkinsonism, Narcotic-analgetics, Analgetic-antipyretics, Psychopharmacological drugs, diagnostica sex hormones allergens, antifungal agents, Chronic Obstructive Pulmonary Disease (COPD) or any combination thereof.

In an embodiment of the invention, said active ingredient is selected from the group consisting of: ace-inhibitors, antianginal drugs, antiarrhythrmas, anti-asthmatics, anti-cholesterolemics, analgesics, anesthetics, anticonvulsants, anti-depressants, antidiabetic agents, anti-diarrhea preparations, antidotes, anti-histamines, anti-hypertensive drugs, anti-inflammatory agents, anti-lipid agents, antimanics, anti-nauseants, anti-stroke agents, anti-thyroid preparations, anti-tumor drugs, anti-viral agents, acne drugs, alkaloids, amino acid preparations, anti-tussives, antiuricemic drugs, anti-viral drugs, anabolic preparations, systemic and non-systemic antiinfective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, .endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, inmosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocryptine or nicotine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

In an embodiment of the invention, said active ingredient is selected from the group consisting of anti-histamines, decongestants, smoking cessation aids, diabetes II agents, or any combination thereof.

Preferred pharmaceuticals to be used are anti-histamines such as cetirizine, decongestants such as phenylephrine, smoking cessation aids such as nicotine salts and diabetes II agents such as metformin.

In an embodiment of the invention, said active ingredient is selected from the group consisting of metformin, cetirizine, levo cetirizine, phenylephrine, flurbiprofen, nicotine, nicotine bitartrate, nicotine polacrilex, nicotine in combination with alkaline agents, nicotine in combination with caffeine, sodium picosulfate, fluor, fluor in combination with fruit acids, chlorhexidine, or any derivatives thereof, salts thereof, isomers thereof, nicotine antagonists, combinations thereof or compounds comprising one or more of these. In the example provided herein nicotine was provided as nicotine polacrilex, which could optionally be combined with buffers.

Further useful nicotine combinations may be nicotine phthalate, nicotine sulphate, nicotine tartrate, nicotine citrate, and nicotine lactate. Alkaline agents to combine with nicotine may be alkalimetal carbonates and bicarbonates such as sodium carbonate and sodium bicarbonate.

An example of a fruit acid to combine with fluor may be malic acid.

In an embodiment of the invention, said active ingredient is selected from the group consisting of ephedrine, pseudo ephedrine, caffeine, loratadine, sildenafil, simvastatin, sumatriptan, acetaminophen, calcium carbonate, vitamin D, ibuprofen, aspirin, alginic acid in combination with aluminum hydroxide and sodium bicarbonate, ondansetron, Tibolon, Rimonabant, Varenicline, allergenes, sitagliptin or any derivatives thereof, salts thereof, isomers thereof, combinations thereof or compounds comprising one or more of these.

In an embodiment of the invention, calcium carbonate may be combined with vitamin D, and in another embodiment of the invention, aspirin may be combined with vitamin C.

In an embodiment of the invention, said active ingredient is selected from the group consisting of phytochemicals, such as resveratrol and anthocyanine; herbals, such as green tea or thyme; antioxidants, such as polyphenols; micronutrients; mouth moisteners, such as acids; throat soothing ingredients; appetite suppressors; breath fresheners, such as zinc compounds or copper compounds; diet supplements; cold suppressors; cough suppressors; vitamins, such as vitamin A, vitamin C or vitamin E; minerals, such as chromium; metal ions; alkaline materials, such as carbonates; salts; herbals, dental care agents, such as remineralisation agents, antibacterial agents, anticaries agents, plaque acid buffering agents, tooth whiteners, stain removers or desensitizing agents; and combinations thereof.

In an embodiment of the invention, said active ingredient comprises a combination of a metal salt, such as a zinc salt or a cobber salt, and vitamin C.

In an embodiment of the invention, a zinc salt and vitamin C may be combined with pectin and a cooling or warming agent. According to a preferred embodiment of the invention, the zinc salt is zinc gluconate.

In an embodiment of the invention, said active ingredient is co-enzyme Q10.

In an embodiment of the invention, said active ingredient is a combination of a calcium salt and vitamin D.

In a preferred embodiment of the invention, the calcium salt is calcium carbonate. According to a preferred embodiment of the invention, the vitamin D is vitamin D3.

In an embodiment of the invention, said active ingredient is a combination of a chromium compound and green coffee bean extract.

According to a preferred embodiment of the invention, the chromium compound is chromium picolinate. In an embodiment of the invention, the combination of a chromium compound and green coffee bean extract may be further combined with green tea extract.

In an embodiment of the invention, said active ingredient is Omega-3.

In an embodiment of the invention, said active ingredient is selected from the group consisting of di-peptides, tri-peptides, oligo-peptides, deca-peptides, deca-peptide KSL, deca-peptide KSL-W, amino acids, proteins, or any combination thereof.

In an embodiment of the invention, said active ingredient comprises a probiotic bacteria, such as lactobacilli, bifidobacteria, lactococcus, streptococcus, leuconostoccus, pediococcus or enterococcus.

In an embodiment of the invention, said active ingredient comprises a prebiotic, such as fructose, galactose, mannose, insulin or soy.

In an embodiment of the invention, said compressed chewing gum comprises a center-fill.

In an embodiment of the invention, said center-fill is a liquid, a semi-liquid, or a solid composition.

In an embodiment of the invention, said center-fill is a solid or semi-liquid composition in combination with one or more enzymes suitable for enzymatic liquification of said solid or semi-liquid.

In an embodiment of the invention, said center-fill comprises bulk sweetener, high-intensity sweetener, flavor, active ingredients, pharmaceutical ingredients and combinations thereof.

In a preferred center-fill embodiment the center-fill comprises maltitol syrup optionally in combination with polyol syrups.

In an embodiment of the invention, a compressed chewing gum according to any of the claims 1-14 comprises one or more encapsulation delivery systems.

In an embodiment of the invention, said one or more encapsulation delivery systems comprise at least one encapsulating material and at least one ingredient encapsulated within said encapsulating material.

In an embodiment of the invention, at least one of said encapsulating material is a copolymer.

In an embodiment of the invention, at least one of said encapsulation material comprises PVA.

In some embodiments, encapsulating material used in a delivery system may include one or more of the following: polyvinyl acetate, polyethylene, crosslinked polyvinyl pyrrolidone, polymethylmethacrylate, polylactidacid, polyhydroxyalkanoates, ethylcellulose, polyvinyl acetatephthalate, polyethylene glycol esters, methacrylicacid- co-methylmethacrylate, ethylene- vinylacetate (EVA) copolymer, and the like, and combinations thereof.

In some embodiments, encapsulating material used in a delivery system may include one or more of the following: hydroxypropylmethylcellulose (HPMC), methylcellulose (MC), hydroxyetylcellulose (HEC), hydroxypropylcellulose (HPC), metacrylate aminoester copolymer, metacrylate ester copolymers metacrylate acid copolymers, cellulose acetate phthalate (CAP), shellac, cellulose acetate trimelliate (CAT), hydroxypropyl metylcellulose phthalate (HPMCP), Zein and the like, and combinations thereof.

In an embodiment of the invention, at least one of said encapsulation material comprises gelatine.

In an embodiment of the invention, at least one of said encapsulation material is selected from the group consisting of natural resin, such as a polyterpene resin, hydrogenated vegetable oil, wax, and combinations thereof.

In an embodiment of the invention, at least one of said encapsulation material comprises natural resin and PVA.

In an embodiment of the invention, at least one of said encapsulation material comprises an active ingredient.

In an embodiment of the invention, said chewing gum granules comprises biodegradable gum base.

In an embodiment of the invention, said biodegradable gum base comprises at least one biodegradable polyester polymer.

In an embodiment of the invention, said biodegradable gum base comprises at least one biodegradable elastomer.

In an embodiment of the invention, said at least one biodegradable polyester polymer is obtained from polymerization of at least one cyclic ester selected from the group consisting of lactide, glycolide, trimethylene carbonate, δ-valerolactone, β-propiolactone and ε-caprolactone, and polyesters obtained by polycondensation of a mixture of open-chain polyacids and polyols, for example, adipic acid and di(ethylene glycol), or any combination thereof.

In an embodiment of the invention, said biodegradable gum base comprises at least one polymer selected from the group consisting of polyesters, poly(ester-carbonates), polycarbonates, polyester amides, polyhydroxy alkanoates, polypeptides, homopolymers of amino acids such as polylysine, proteins such as prolamin, and protein derivatives such as protein hydrolysates including a zein hydrolysate, or any combination thereof.

In an embodiment of the invention, said chewing gum granules are substantially free of non-biodegradable polymers.

In an embodiment of the invention, said compressed chewing gum is provided with an outer coating.

In an embodiment of the invention, said outer coating is selected from the group consisting of hard coating, soft coating and edible film-coating or any combination thereof.

In an embodiment of the invention, the outer coating comprises at least one additive component selected from the group consisting of a binding agent, a moisture absorbing component, a film forming agent, a dispersing agent, an anti-sticking component, a bulking agent, a flavoring agent, a coloring agent, a pharmaceutically or cosmetically active component, a lipid component, a wax component, a sugar, an acid and an agent capable of accelerating the after-chewing degradation of the degradable polymers or any combination thereof.

In an embodiment of the invention, said compressed chewing gum comprises coating in an amount of 0.1 to 95% by weight of a coated chewing gum piece, preferably 0.1 to 75% by weight of a coated chewing gum piece.

Moreover, the invention relates to a chewing gum granule according to any of the claims 1-14, wherein said free-flowing agent is an anti-agglomeration agent.

Moreover the invention relates to a method for producing chewing gum granules according to any of the claims 1-14, which method comprises at least the steps of:
a) feeding a gum composition into an extruder,
b) pressurizing the gum composition in the extruder, and
c) extruding the gum composition through a die means.

The gum composition is under pressure in the extruder and moreover the composition and/or the die plate are heated to obtain a satisfying result. Directly following the cutting into the chewing gum granules a cooling of the granules is performed to make the granules form-stable, and during this process these are form-stabilized into ball-shaped pellets.

In an embodiment of the invention, said method further comprises the step of cutting the extruded gum composition during cooling by liquid.

In an embodiment of the invention, said method further comprises the step of cutting the extruded gum composition during cooling by air.

Moreover, the invention relates to the use of chewing gum granules according to any of the claims 1-14 in a chewing gum product.

Moreover, the invention relates to a chewing gum granule comprising gum base, said chewing gum granule being substantially ball-shaped, said chewing gum granule having an average diameter below 1900 µm, and the surface of said chewing gum granule being provided with a free-flowing agent comprising active ingredients

According to an embodiment of the invention, active ingredients are distributed on the surface of said chewing gum granule as a part of another free-flowing agent or possibly the active ingredients constitutes the free-flowing agent solely.

By using a mixture of free-flowing agent and active ingredient it is obtained that a desired concentration of active ingredient in free-flowing agent can be used to powder the surface and at the same time avoid that the addition of active ingredient on the surface will result in less satisfactory properties.

According to an embodiment of the invention, active ingredients may provide good properties similar to other free-flowing agents mentioned herein.

In an embodiment of the invention, said active ingredients are one or more of the active ingredients.

In an embodiment of the invention, chewing gum granule being substantially ball-shaped, said chewing gum granule having an average diameter below 1900 µm, and the surface of said chewing gum granule being provided with a free-flowing agent comprising a polyol.

In an embodiment of the invention, the surface of said chewing gum granule being provided with 0.01 to 70% by weight of the polyol.

According to an embodiment of the invention, the surface of the chewing gum granule may be provided with 0.01 to 99% by weight of a sweetener such as a sugar or a polyol.

According to an embodiment of the invention, the surface of the chewing gum granule may be provided with up to 100% by weight of a sweetener such as a sugar or a polyol.

According to an embodiment of the invention, the surface of the chewing gum granule is provided with 0.01 to 99% by weight of a saccharide.

Moreover, the invention relates to a chewing gum granule containing gum base, said chewing gum granule being substantially ball-shaped, and the surface of said chewing gum granule being provided with at least one polyol.

In an embodiment of the invention, the free-flowing agent is attached to said chewing gum granule by means of dusting, spraying, electrostaticity or agglomeration or combinations thereof.

In an embodiment of the invention, the free-flowing agent is one or more of the free-flowing agents according to claim 14.

In an embodiment of the invention, said chewing gum granule comprise active ingredients.

According to an embodiment of the invention, active ingredients may be present as distributed on the surface of said chewing gum granule and for the same granule also as a component of the granule body.

In an embodiment of the invention, said active ingredients are comprised in particles, and wherein the average diameter of said particles is less than 50% of the average diameter of said chewing gum granules.

In order to obtain satisfying results the average diameter of said particles should be less than 10% or even smaller in order to ensure that dosing of the active ingredients can be performed very accurately without risking that one particle more or less will hugely modify the dose given per granule.

Moreover, the invention relates to a chewing gum granule , wherein said chewing gum granule is a chewing gum granule according to any of the claims 1-14.

### THE DRAWINGS

The invention will now be described in more detail with reference to the drawings of which:
fig. 1a shows a cross-sectional view of a spherically ball-shaped chewing gum granule,
figs. 1b and 1c show a cross-sectional view of a spherically ball-shaped chewing gum granule having free-flowing agent provided on the surface,
fig. 2a-2e show cross-sectional views of different embodiments of substantially ball-shaped chewing gum granules according to the invention,
fig. 3a-3c show cross-sectional views of different compositions according to the invention,
fig. 4a shows a photo of a composition of chewing gum granules according to the invention, and
fig. 4b shows a photo of a composition of grinded prior art chewing gum granules. fig. 5a shows a chewing gum built up of 2 modules,
fig. 5b shows a chewing gum built up of 3 modules,
fig. 6a-6b show charts of different module content of chewing gum ingredients.

### DETAILED DESCRIPTION

In the prior art anti-agglutination agent has been attached to chewing gum granules in order to avoid sticking to other granules. For granules smaller than about 2.0 mm in diameter the amount of anti-agglutination agent needed has resulted in texture and disintegration problems of the compressed chewing gum.

The term "anti-agglutination agent" is used in prior art to indicate the objective for applying anti-agglutination agent, i.e. preventing agglutination of the granules. However, a problem with difficulties in handling the granules may still be present in spite of a decreased agglutination of the granules.

Therefore, the objective is larger than in prior art and according to the present invention favorable abilities regarding minimizing agglutination and increasing flowing abilities of the granules have been seen. Therefore, the term "free-flowing agent" is used in this document to indicate a further functionality of the agent used even though an overlap may be seen between which agents are used as anti-agglutination agent in prior art and which are used as free-flowing agent according to the present invention.

Initially mechanical and physical properties of the granule according to an embodiment of the invention will be discussed and subsequently in the description the composition of gum base and chewing gum will be discussed.

According to the present invention it has been established that a chewing gum granule according to the invention with an average diameter below 1900 µm and with between 0.5 and 18% by weight of free-flowing agent provided on the surface of said granule holds satisfying stability and texture, possesses increased flowing abilities, and minimizes agglutination of the granules. With increased flowing abilities improvements in transportation and handling of the granules is obtained.

The term "compressed chewing gum" is used in this document to indicate a chewing gum manufactured by compressing granules and optionally other ingredients at a certain pressure to obtain a chewing gum. The term "tabletting" used in this document is synonymous with compressing, whereas the term tabletting in prior art sometimes indicate the process of making standard chewing gum pieces (tablets) by punching or the like.

The term "average diameter" as used herein is defined as the diameter of a sphere having the same volume as the granule. Although the granules produced according to the present invention mostly are substantially ball-shaped, variations in shape may occur, and according to the definition granules having the same volume also have the same average diameter.

The term "mean value diameter" as used herein is defined as the arithmetic mean value of the average diameters of a number of granules or particles in a composition. Unless otherwise indicated all percentages are % by weight.

Unless otherwise indicated, as used herein with regard to polymers, the term "molecular weight" means number average molecular weight (Mn) in g/mol.

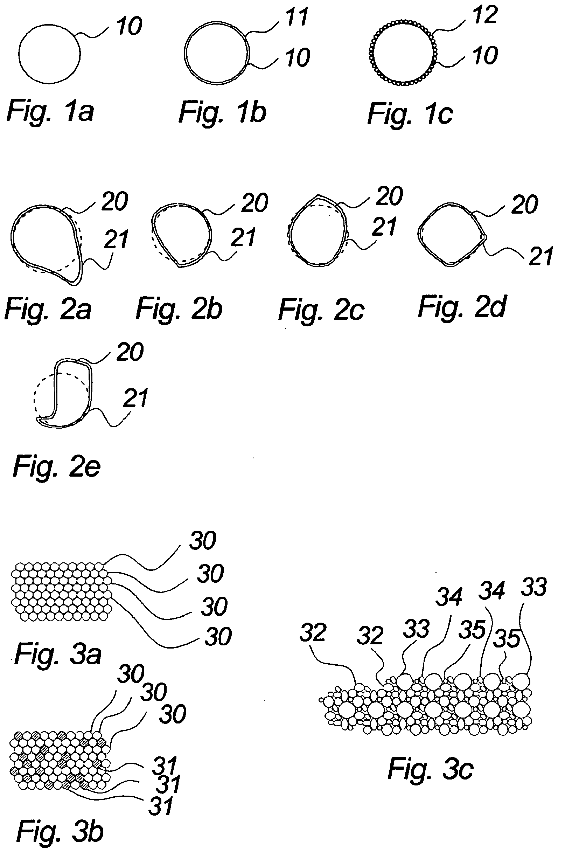

In fig. 1a is shown a spherically ball-shaped chewing gum granule 10 according to an embodiment of the invention. In fig. 1b is shown a first embodiment of a granule 10 of fig. 1a on which is provided a layer of free-flowing agent 11. Although the layer of free-flowing agent in fig. 1b is indicated to be strictly ball-shaped, this is mainly for indicative purpose as the actual look of the granule with free-flowing agent will depend on the relative sizes between the granule and the free-flowing agent and the shaping of the actual free-flowing agent. In fig. 1c is shown a second embodiment of a granule 10 provided with a layer of free-flowing agent 12. In this case the free-flowing agent is substantially ball-shaped and the ratio between the diameter of free-flowing agent and granule is as high as approx. 1:15. The reason for showing an example with a ratio as high as this, is to show how the free-flowing agent may be distributed on the surface of the granule. In this specific case the distribution is homogeneous, however, a layer of free-flowing agent on the surface of the granule need not be homogenously distributed at all and small or large holes in the free-flowing agent layer may occur as well. What is important is that a good distribution of the free-flowing agent is obtained and that the amount of free-flowing agent is not too high, preferably that a minimal amount of free-flowing agent is used.

By means of granules according to the invention a compressed chewing gum may be obtained which possesses a crunch feel during chewing.

The chewing gum granule preferably has an average diameter below 1900 µm, whereby more satisfying properties are achieved regarding the compressed chewing gum. Too large granules may result in a missing "crunch" feel and a precise dosing of weight per chewing gum may turn out to be more difficult.

By application of a free-flowing agent on the surface of the granules as e.g. shown in figs. 1b and 1c an improvement is obtained in reducing the sticking of one granule to another, i.e. the static interaction between granules. To further ensure good flowing properties when the free-flowing agent is attached to the surface of the granules, i.e. the dynamic interaction between granules, the shaping of the granules is important in that ball-shaped granules need less free-flowing agent in order to possess satisfying properties as compared to non-ball-shaped granules.

What is meant within the scope of the invention with the term "ball-shaped" will be apparent when looking at the granules 20 in figs. 2a-2e, which are all ball-shaped within the scope of the invention, i.e. having a shape having at least a part of the surface which resembles the smooth surface of a ball and preferably with the major part of the surface being smooth. All granules 20 in figs. 2a-2e are provided with a layer of free-flowing agent 21, which in the same way as for figs. 1b and 1c may or may not be as smoothly distributed as indicated in the figures.

In the figs. 2a-2e a spherical ball-shape is dashed to indicate how the average diameter of each granule is found. The average diameter of a granule is defined as the diameter of a spherical ball-shape having the same volume as the granule.

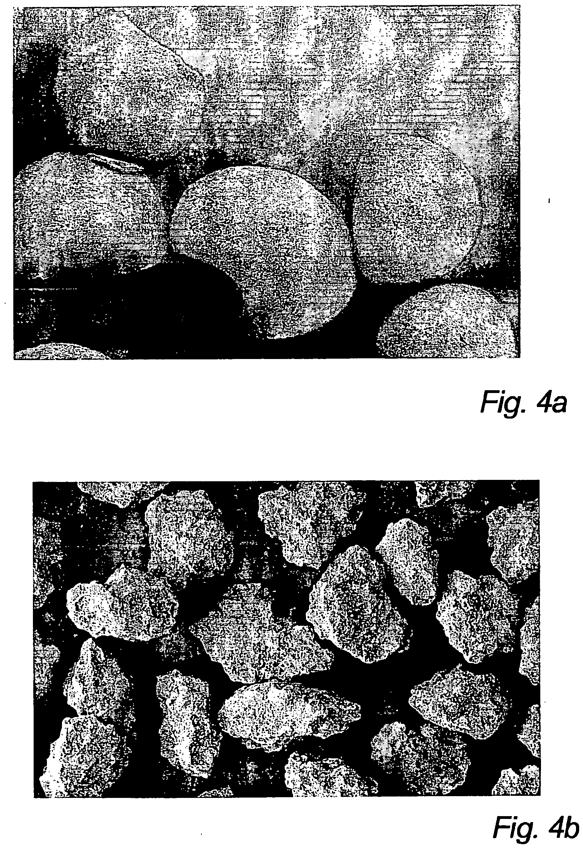

Further to explain the meaning of ball-shaped, fig. 4a is a photo of ball-shaped granules according to various embodiments of the invention, here with a layer of CaCO₃ provided on the surface as free-flowing agent, and fig. 4b is a photo of prior art granules, which are not ball-shaped. The difference between the two kinds of granules is striking and the need for a higher amount of free-flowing agent on the prior art granules to ensure no sticking should be apparent i.e. due to the highly increased surface area. Possibly the disadvantageous shaping of the prior art granules may be due to a manufacturing process involving cooling and crushing of the chewing gum to obtain the shown granules.

Substantially ball-shaped granules are especially advantageous in that they flow better and they do not lock each other mechanically as may be seen for the prior art granules. In order to prevent the risk of mechanically locking of prior art granules very high amounts of free-flowing agent should be added, which would be bad for the texture of the granule and might cause disintegration of the same. The substantially ball-shaped granules are therefore in particular advantageous in extruding, cutting and compressing processes.

Figs. 3a-3c illustrate compositions of granules according to embodiments of the invention. In fig. 3a the composition consists of chewing gum granules 30 uniformly shaped and sized. Fig. 3c illustrates a composition of chewing gum granules 32, 33, 34, 35 with varying shapes and sizes.

A composition of a mixture of chewing gum granules and tablet material might as well look as fig. 3c, in which e.g. 32 and 33 could be chewing gum granules and e.g. 34 and 35 could be tablet material.

Fig. 3b illustrates a composition comprising a mixture of chewing gum granules 30 and tablet material 31, wherein the size of the chewing gum granules 30 and the tablet material 31 is essentially the same.

Figs. 3a-3c are illustrative examples only of how compositions may look according to embodiments of the invention. As should be apparent from the content of the description, the compositions as mentioned herein may comprise ball-shaped chewing gum granules with a variety of average diameters alone or in combination with tablet material with a variety of average diameters and/or active ingredients.

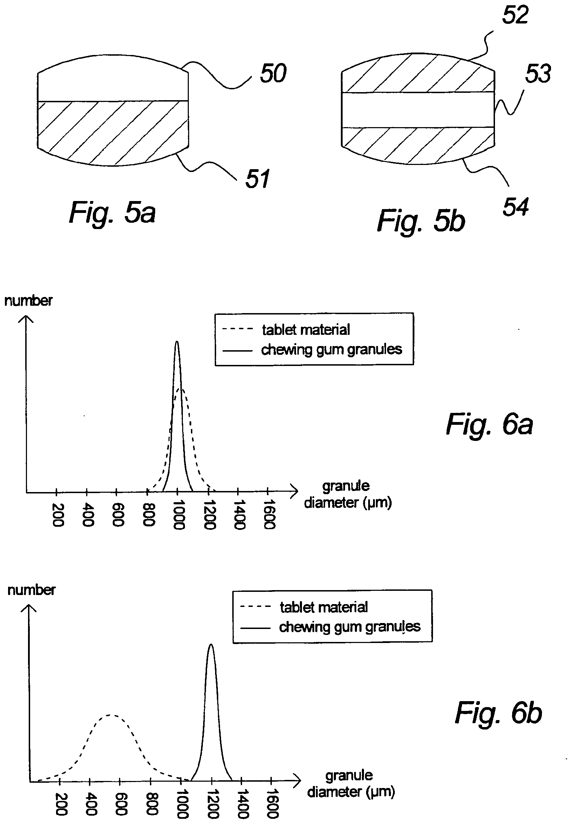

Figs. 5a and 5b illustrate two examples of multi-layer chewing gums according to embodiments of the invention. Fig. 5a illustrates an embodiment wherein a chewing gum module 51 and a tablet module 50 constitute a two-module chewing gum tablet. Fig. 5b illustrates another embodiment wherein two chewing gum modules 52, 54 are placed on each side of a central tablet module 53.

Figs. 5a and 5b are illustrative examples only of how a multi-layer chewing gum according to embodiments of the invention may be built. Any other combination with tablet modules, chewing gum modules, both with or without active ingredients as described in the description is within the scope of the invention.

Figs. 6a and 6b illustrate typical distributions of chewing gum granules and tablet material in a composition or module with granule diameter (average diameter) along the x-axis and number of granules/tablet material with a certain average diameter along the y-axis. Fig. 6a illustrates a composition wherein the mean value diameter of the chewing gum granules and of the tablet material is approximately comparable. This is in contrast to the embodiment shown in fig. 6b, wherein the chewing gum granules has a mean value diameter around 1200 µm and the tablet material has a mean value diameter around 550 µm.

Figs. 6a and 6b are illustrative examples only of how a distribution of chewing gum granule sizes and tablet material sizes according to embodiments of the invention may be. Numerous other distributions may be within the scope of the invention, but preferably the size distribution of chewing gum granules is not too broad.

Due to the broad distribution of average diameter size, a mixture as indicated in fig. 6b will be much more likely to cause segregation of the mixture and thereby cause inhomogeneities in the final product if not carefully mixed immediately prior to compression.

The formulations, manufacturing processes and combinations thereof given herein are exemplary and only given for the purpose of evaluating and explaining different features of the invention. Manufacturing processes and formulations may be varied significantly within the scope of the invention. Specific variations and details with respect to ingredients, formulations and manufacturing processes within the scope of the invention are given below.

In accordance with the general principles in manufacturing a chewing gum and a chewing gum granule within the scope of the invention, variations of different suitable ingredients are listed and explained below.

Chewing gum of the present invention typically comprises a water-soluble portion, a water-insoluble chewable gum base portion and flavouring agents. The water-soluble portion dissipates with a portion of the flavouring agent over a period of time during chewing. The gum base portion is retained in the mouth throughout the chew. The term chewing gum refers to both a chewing and bubble type gum in its general sense.

The gum base is the masticatory substance of the chewing gum, which imparts the chew characteristics to the final product. The gum base typically defines the release profile of flavors and sweeteners and plays a significant role in the gum product.

The insoluble portion of the gum typically may contain any combination of elastomers, vinyl polymers, elastomer plasticizers, waxes, softeners, fillers and other optional ingredients such as colorants and antioxidants.

The composition of gum base formulations can vary substantially depending on the particular product to be prepared and on the desired masticatory and other sensory characteristics of the final product. However, typical ranges (% by weight) of the above gum base components are: 5 to 80% by weight elastomeric compounds, 5 to 80% by weight elastomer plasticizers, 0 to 40% by weight of waxes, 5 to 35% by weight softener, 0 to 50% by weight filler, and 0 to 5% by weight of miscellaneous ingredients such as antioxidants, colourants, etc. The gum base may comprise about 5 to about 95 percent, by weight, of the chewing gum, more commonly, the gum base comprises 10 to about 60 percent of the gum.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types.

Elastomer plasticizers vary the firmness of the gum base. Their specificity on elastomer inter-molecular chain breaking (plasticizing) along with their varying softening points cause varying degrees of finished gum firmness and compatibility when used in base. This may be important when one wants to provide more elastomeric chain exposure to the alkanic chains of the waxes.

The elastomer compounds may be of natural origin but are preferably of synthetic origin, preferably synthetic polyesters.

It is noted that gum base or gum granules may also include components typically referred to as chewing gum ingredients.

The chewing gum may, according to embodiments of the invention, comprise conventionally non-biodegradable polymers, such as natural resins, synthetic resins and/or synthetic or natural elastomers.

According to an embodiment of the invention, at least a part of the polymers of the chewing gum are biodegradable.

In an embodiment of the invention, the chewing gum may comprise combinations of biodegradable polymers and polymers generally regarded as non-biodegradable, such as natural resins, synthetic resins and/or synthetic/natural elastomers.

In an embodiment of the invention, said natural resin comprises terpene resins, e.g. derived from alpha-pinene, beta-pinene, and/or d-limonene, natural terpene resins, glycerol esters of gum rosins, tall oil rosins, wood rosins or other derivatives thereof such as glycerol esters of partially hydrogenated rosins, glycerol esters of polymerized rosins, glycerol esters of partially dimerised rosins, pentaerythritol esters of partially hydrogenated rosins, methyl esters of rosins, partially hydrogenated methyl esters of rosins or pentaerythritol esters of rosins and combinations thereof.

Materials to be used for the above-mentioned encapsulation methods might e.g. include Gelatine, Wheat protein, Soya protein, Sodium caseinate, Caseine, Gum arabic, Mod. starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

Examples of generally non-biodegradable synthetic resins include polyvinyl acetate, vinyl acetate-vinyl laurate copolymers and mixtures thereof. Examples of non-biodegradable synthetic elastomers include, but are not limited to, synthetic elastomers listed in Food and Drug Administration, CFR, Title 21, Section 172,615, the Masticatory Substances, Synthetic) such as polyisobutylene. e.g. having a gel permeation chromatography (GPC) average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3:1, polyvinyl acetate (PVA), e.g. having a GPC average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer, and combinations hereof.

The elastomers (rubbers) employed in the gum base may vary depending upon various factors such as the type of gum base desired, the texture of gum composition desired and the other components used in the composition to make the final chewing gum product. The elastomer may be any water-insoluble polymer known in the art, and includes those gum polymers utilized for chewing gums and bubble gums. Illustrative examples of suitable polymers in gum bases include both natural and synthetic elastomers. For example, those polymers which are suitable in gum base compositions include, without limitation, natural substances (of vegetable origin) such as chicle gum, natural rubber, crown gum, nispero, rosidinha, jelutong, perillo, niger gutta, tunu, balata, guttapercha, lechi capsi, sorva, gutta kay, and the like, and mixtures thereof. Examples of synthetic elastomers include, without limitation, styrene-butadiene copolymers (SBR), polyisobutylene, isobutylene-isoprene copolymers, polyethylene, polyvinyl acetate and the like, and mixtures thereof.

It is common in the industry to combine in a gum base a synthetic elastomer having a high molecular weight and a synthetic elastomer having a low molecular weight. Examples of such combinations are polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Examples of natural resins are: Natural rosin esters, often referred to as ester gums including as examples glycerol esters of partially hydrogenated rosins, glycerol esters of polymerised rosins, glycerol esters of partially dimerized rosins, glycerol esters of tally oil rosins, pentaerythritol esters of partially hydrogenated rosins, methyl esters of rosins, partially hydrogenated methyl esters of rosins, pentaerythritol esters of rosins, synthetic resins such as terpene resins derived from alpha-pinene, beta-pinene, and/or d-limonene, and natural terpene resins.

The chewing gum according to the invention may be provided with an outer coating. The applicable hard coating may be selected from the group comprising of sugar coating and a sugarless coating and a combination thereof. The hard coating may e.g. comprise 50 to 100% by weight of a polyol selected from the group consisting of sorbitol, maltitol, mannitol, xylitol, erythritol, lactitol and Isomalt and variations thereof. In an embodiment of the invention, the outer coating is an edible film comprising at least one component selected from the group consisting of an edible film-forming agent and a wax. The film-forming agent may e.g. be selected from the group comprising cellulose derivative, a modified starch, a dextrin, gelatine, shellac, gum arabic, zein, a vegetable gum, a synthetic polymer and any combination thereof. In an embodiment of the invention, the outer coating comprises at least one additive component selected from the group comprising of a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, an acid and an agent capable of accelerating the after-chewing degradation of the degradable polymer.

Generally, the ingredients may be mixed by first melting the gum base and adding it to the running mixer. Colors, active agents and/or emulsifiers may also be added at this time. A softener such as glycerin may also be added at this time, along with syrup and a portion of the bulking agent/sweetener. Further portions of the bulking agent/sweetener may then be added to the mixer. A flavoring agent is typically added with the final portion of the bulking agent/sweetener. A high-intensity sweetener is preferably added after the final portion of bulking agent and flavor has been added.

The entire mixing procedure typically takes from five to fifteen minutes, but longer mixing times may sometimes be required. Those skilled in the art will recognize that many variations of the above-described procedure may be followed. Including the one-step method described in US patent application 2004/0115305 hereby incorporated as reference. Chewing gums are formed by extrusion, compression, rolling and may be centre filled with liquids and/or solids in any form.

The chewing gum according to the present invention may also be provided with an outer coating, which may be a hard coating, a soft coating, a film coating, or a coating of any type that is known in the art, or a combination of such coatings. The coating may typically constitute 0.1 to 75% by weight of a coated chewing gum piece.

One preferred outer coating type is a hard coating, which term is including sugar coatings and sugar-free (or sugarless) coatings and combinations thereof. The object of hard coating is to obtain a sweet, crunchy layer, which is appreciated by the consumer and to protect the gum centers. In a typical process of providing the chewing gum centers with a protective sugar coating the gum centers are successively treated in suitable coating equipment with aqueous solutions of crystallizable sugar such as sucrose or dextrose, which, depending on the stage of coating reached, may contain other functional ingredients, e.g. fillers, colors, etc.

In one presently preferred embodiment, the coating agent applied in a hard coating process is a sugarless coating agent, e.g. a polyol including as examples sorbitol, maltitol, mannitol, xylitol, erythritol, lactitol and isomalt or e.g. a mono- di-saccharide including as example trehalose.

Or alternatively a sugar-free soft coating e.g. comprising alternately applying to the centers a syrup of a polyol or a mono- di-saccharide, including as examples sorbitol, maltitol, mannitol, xylitol, erythritol, lactitol, isomalt and trehalose.

In further useful embodiments, a film coating is provided by film-forming agents such as a cellulose derivative, a modified starch, a dextrin, gelatine, zein, shellec, gum arabic, a vegetable gum, a synthetic polymer, etc. or a combination thereof.

In an embodiment of the invention, the outer coating comprises at least one additive component selected from the group comprising a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, and an acid.

A coated chewing gum center according to the invention may have any form, shape or dimension that permits the chewing gum center to be coated using any conventional coating process.

It should however be noted that application of different coating should be done with care as compressed chewing gum tablets may be very affected by direct contact with moisture or water.

The glass transition temperature (T_{g}) may be determined by for example DSC (DSC: differential scanning calorimetry). The DSC may generally be applied for determining and studying of the thermal transitions of a polymer and specifically, the technique may be applied for the determination of a second order transition of a material, i.e. a thermal transition that involves a change in heat capacity, but does not have a latent heat. The glass transition is a second-order transition.

The composition of gum base formulations can vary substantially depending on the particular product to be prepared and on the desired masticatory and other sensory characteristics of the final product. However, typical ranges of the above gum base components are: 5 to 80% by weight of elastomeric compounds, 5 to 80% by weight of elastomer plasticizers, 0 to 40% by weight of waxes, 5 to 35% by weight of softener, 0 to 50% by weight of filler, and 0 to 5% by weight of miscellaneous ingredients such as antioxidants, colorants, etc. The gum base may comprise about 5 to about 95% by weight of the chewing gum, more commonly; the gum base comprises 10 to about 60% by weight of the gum.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types.

Elastomer plasticizers vary the firmness of the gum base. Their specificity on elastomer inter-molecular chain breaking (plasticizing) along with their varying softening points cause varying degrees of finished gum firmness and compatibility when used in gum base. This may be important when one wants to provide more elastomeric chain exposure to the alkanic chains of the waxes.

If desired, conventional elastomers or resins may be supplemented or substituted by biodegradable polymers.

Biodegradable polymers that may be used in the chewing gum of the present invention may be homopolymers, copolymers or terpolymers, including graft- and block-polymers.

Useful biodegradable polymers, which may be applied as gum base polymers in the chewing gum of the present invention, may generally be prepared by step-growth polymerization of di-, tri- or higher-functional alcohols or esters thereof with di-, tri-or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed.

The usually preferred polyfunctional alcohols contain 2 to 100 carbon atoms as for instance polyglycols and polyglycerols.

Gum base polymers may both be resinous and elastomeric polymers.

In the polymerization of a gum base polymer for use in the chewing gum of the present invention, some applicable examples of alcohols, which may be employed as such or as derivatives thereof, include polyols such as ethylene glycol, 1,2-propanediol, 1,3-propanediol, 1,3-butanediol, 1,4-butanediol, 1,6-hexanediol, diethylene glycol, 1,4-cyclohexanediol, 1,4-cyclohexanedimethanol, neopentyl glycol, glycerol, trimethylolpropane, pentaerythritol, sorbitol, mannitol, etc.

Suitable examples of environmentally or biologically degradable chewing gum base polymers, which may be applied in accordance with the gum base of the present invention, include degradable polyesters, polycarbonates, polyester amides, polyesterurethanes, polyamides, prolamine, polypeptides, homopolymers of amino acids such as polylysine, and proteins including derivatives hereof such as e.g. protein hydrolysates including a zein hydrolysate.

Polyesters which may be applied in accordance with the gum base of the present invention may e.g. be as seen in EP 1 545 234 or EP 0 711 506 as incorporated herein by reference.

Further polymers which may be used in the gum base according to embodiments of the invention comprise:
enzymatically hydrolyzed zein, plasticized poly(D,L-lactic acid) and poly(D,L-lactic acid-co-glycolic acid), polyhydroxyalkanoates having side chain lengths of C₄ to C₃₀, poly(lactic acid) copolymers selected from the group consisting of poly(lactic acid-dimer fatty acid-oxazoline) copolymers and poly(lactic acid-diol-urethane) copolymers,
at least one polyester wherein the polyester includes monomers selected from the group consisting of lactic acid, lactide, glycolic acid, glycolide, citric acid, adipic acid, caprolactone, ethylene oxide, ethylene glycol, propylene oxide, and propylene glycol, and combinations thereof,
at least one polyester that is produced through a reaction of glycerol and at least one acid chosen from the group consisting of citric acid, fumaric acid, adipic acid, malic acid, succinic acid, suberic acid, sebacic acid, dodecanedioic acid, glucaric acid, glutamic acid, glutaric acid, azelaic acid, and tartaric acid,
at least one polyester that is produced through a reaction of at least one alcohol chosen from the group consisting of glycerol, propylene glycol, and 1,3 butylene diol, and at least one acid chosen from the group consisting of fumaric acid, adipic acid, malic acid, succinic acid, and tartaric acid, the polyester being end-capped with a monofunctional ingredient selected from the group consisting of alcohols, acids, chlorides, and esters,
and further such as can be found in e.g. US 6,773,730, US 6,613,363, US 6,194,008, US 5,580,590, US 6,858,238, US 6,017,566, US 6,013,287, and US 5,800,848, which are all hereby incorporated by reference.

The polyesters formed on the basis of di- or polyfunctional acids and di- or polyfunctional alcohols may be produced according to known methods, one of which includes US2007/043200, hereby incorporated by reference.

The prolamine may e.g. be selected from the group consisting of zein, corn gluten meal, wheat gluten, gliadin, glutenin and any combination thereof. Methods of providing such a polymer are disclosed in US2004/001903, hereby incorporated by reference.

Examples of such protein based compounds include but are not limited to prolamine, zein, corn gluten meal, wheat gluten, gliadin, glutenin and combinations thereof.

Such suitable biodegradable gum base polymers include polyesters, polycarbonates, polyesteramides, polyesterurethanes, polyamides, prolamine, and combinations thereof.

Polycarbonates may typically be co-polymerised with polyesters. Some typically preferred cyclic carbonates, which may be used as starting material, may e.g. comprise trimethylene carbonate, 2,2-dimethyltrimethylene carbonate, 2-methyltrimethylene carbonate, 3-methyltrimethylene carbonate, 2,3-dimethyltrimethylene carbonate, 2,4-dimethyltrimethylene carbonate, 2,3,4-trimethyltrimethylene carbonate, 2,3,3,4-tetramethyltrimethylene carbonate, etc.

In some embodiments, suitable polyesteramides can be constructed from monomers of the following groups: dialcohols, such as ethylene glycol, 1,4-butanediol, 1,3-propanediol, 1,6-hexanediol diethylene glycol and others; and/or dicarboxylic acid, such as oxalic acid, succinic acid, adipic acid and others, including those in the form of their respective esters (methyl, ethyl, etc.); and/or hydroxycarboxylic acids and lactones, such as caprolactone and others; and/or amino alcohols, such as ethanolamine, propanolamine, etc.; and/or cyclic lactams, such as .epsilon.-caprolactam or laurolactam, etc.; and/or .omega.-aminocarboxylic acids, such as aminocaproic acid, etc.; and/or mixtures (1:1 salts) of dicarboxylic acids such as adipic acid, succinic acid etc., and diamines such as hexamethyl enediamine, diaminobutane, etc.

In the case where the polymer mixture is based extensively on thermoplastic starch and an aromatic polyester, an aliphatic-aromatic copolyester, or a polyesteramide, it may be advantageous to add an aliphatic polyester or copolyester, such as polycaprolactone, for example, as a further component. As an example of this there may be mentioned a polymer mixture consisting of thermoplastic starch, at least one polyethylene terephthalate (PET) or a polyalkylene terephthalate, and polycaprolactone. Other examples of aliphatic polyesters or copolyesters are polylactic acid, polyhydroxybutyric acid, polyhydroxybutyric acid-hydroxyvaleric acid copolymer, and/or mixtures thereof.

Suitable polyesters may be obtained through polycondensation polymerisation or ring-opening polymerisation reactions. Some preferred polyesters include those polymerised from at least one carboxylic acid and at least one aliphatic di- or polyfunctional alcohols. The carboxylic acids may include aromatic dicarboxylic acids and aliphatic di- or polyfuncional carboxylic acids. In some preferred embodiments, the majority of the carboxylic acids are aliphatic.

Some of the preferred polyesters according to the invention may e.g. be prepared by step-growth polymerization of di-, tri- or higher-functional alcohols or esters thereof with di-, tri- or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed. Use of branched monomers suppresses the crystallinity of the polyester polymers. Mixing of dissimilar monomer units along the chain also suppresses crystallinity. To control the reaction and the molecular weight of the resulting polymer it is possible to stop the polymer chains by addition of monofunctional alcohols or acids and/or to utilize a stoichiometric imbalance between acid groups and alcohol groups or derivatives of either. Also the adding of long chain aliphatic carboxylic acids or aromatic monocarboxylic acids may be used to control the degree of branching in the polymer and conversely multifunctional monomers are sometimes used to create branching. Moreover, following the polymerization monofunctional compounds may be used to end cap the free hydroxyl and carboxyl groups.

Examples of aliphatic di- or polyfunctional carboxylic acids, which may be applied as monomers of suitable polyesters include oxalic, malonic, citric, succinic, malic, tartaric, fumaric, maleic, glutaric, glutamic, adipic, glucaric, pimelic, suberic, azelaic, sebacic, dodecanedioic acid, etc. Likewise, specific examples of aromatic polyfunctional carboxylic acids may be terephthalic, isophthalic, phthalic, trimellitic, pyromellitic and naphthalene 1,4-, 2,3-, 2,6-dicarboxylic acids and the like. Some preferred polyesters are disclosed in CA2523510, hereby included by reference.

In a preferred embodiment, aliphatic dicarboxylic acids applied in the polyesters are selected from aliphatic dicarboxylic acids having from 4 to 12 carbons, such as succinic acid, glutaric acid, 2-methylglutaric acid, 3-methylglutaric acid, 2,2-dimethylglutaric acid, adipic acid, pimelic acid, suberic acid, azelaic acid and sebacic acid, higher homologues and stereoisomers and mixtures thereof. Preferred aliphatic dicarboxylic acids in this embodiment are succinic acid, glutaric acid, adipic acid, pimelic acid, azelaic acid and sebacic acid, and mixtures thereof.

In an embodiment, aromatic dicarboxylic acids applied in the polyesters contain two carboxyl groups which are bound to one aromatic system. Preferably, the aromatic system is carboaromatic, such as phenyl or naphthyl. In the case of polynuclear aromatics, the two carboxyl groups may be bound to the same ring or different rings. The aromatic system can also have one or more alkyl groups, for example methyl groups. The aromatic dicarboxylic acid is then generally selected from aromatic dicarboxylic acids having from 8 to 12 carbons, such as phthalic acid, isophthalic acid, terephthalic acid, 1,5- and 2,6-naphthalenedicarboxylic acid. Preferred aromatic dicarboxylic acids in this embodiment are terephthalic acid, isophthalic acid and phthalic acid and mixtures thereof.

Furthermore, some usually preferred polyfunctional alcohols suitable for preparing advantageous polyesters according to the invention contain 2 to 100 carbon atoms as for instance polyglycols and polyglycerols. Suitable examples of alcohols, which may be employed in the polymerization process as such or as derivatives thereof, includes polyols such as ethylene glycol, 1,2-propanediol, 1,3-propanediol, 1,3-butanediol, 1,4-butanediol, 1,6-hexanediol, diethylene glycol, 1,4-cyclohexanediol, 1,4-cyclohexanedimethanol, neopentyl glycol, glycerol, trimethylolpropane, pentaerythritol, sorbitol, mannitol, etc. For the purpose of illustration and not limitation, some examples of alcohol derivatives include triacetin, glycerol palmitate, glycerol sebacate, glycerol adipate, tripropionin, etc.

Additionally, with regard to polyesters polymerized from alcohols or derivatives thereof and carboxylic acids or derivatives thereof, chain-stoppers sometimes used are monofunctional compounds. They are preferably either monohydroxy alcohols containing 1-20 carbon atoms or monocarboxylic acids containing 2-26 carbon atoms. General examples are medium or long-chain fatty alcohols or acids, and specific examples include monohydroxy alcohols such as methanol, ethanol, butanol, hexanol, octanol, etc., and lauryl alcohol, myristyl alcohol, cetyl alcohol, stearyl alcohol, stearic alcohol, etc., and monocarboxylic acids such as acetic, lauric, myristic, palmitic, stearic, arachidic, cerotic, dodecylenic, palmitoleic, oleic, linoleic, linolenic, erucic, benzoic, naphthoic acids and substituted napthoic acids, 1-methyl-2 naphthoic acid and 2-isopropyl-1-naphthoic acid, etc.

Typically, an acid catalyst or a transesterification catalyst may be used in such polyester polymerization processes, and non-limiting examples of those are the metal catalysts such as acetates of manganese, zinc, calcium, cobalt or magnesium, and antimony(III)oxide, germanium oxide or halide and tetraalkoxygermanium, titanium alkoxide, zinc or aluminum salts.

In a preferred embodiment of the invention, the polyesters can for example include copolymers containing any combination of the monomers lactic acid, lactide, glycolic acid, glycolide, citric acid, adipic acid, caprolactone, ethylene oxide, ethylene glycol, propylene oxide, propylene glycol and combinations thereof.

Examples of suitable polyesters obtainable by ring-opening polymerization include polyesters comprising combinations of cyclic monomers including the following:
D,L-lactide/ε-caprolactone,
D,L-lactide/TMC
D,L-lactide/δ-valerolactone
D,L-lactide/dioxanone
D,L-lactide

L-lactide/ε-caprolactone
L-lactide/TMC
L-lactide/δ-valerolactone
L-lactide/dioxanone
L-lactide

D,L-lactide/glycolide/ε-caprolactone
D,L-lactide/glycolide/TMC
D,L-lactide/glycolide/δ-valerolactone
D,L-lactide/glycolide/dioxanone
D,L-lactide/glycolide

L-lactide/glycolide/ε-caprolactone
L-lactide/glycolide/TMC
L-lactide/glycolide/δ-valerolactone
L-lactide/glycolide/dioxanone
L-lactide/glycolide

glycolide/ε-caprolactone
glycolide/TMC
glycolide/δ-valerolactone
glycolide/dioxanone
glycolide

D,L-lactide/L-lactide/ε-caprolactone
D,L-lactide/L-lactide/TMC
D,L-lactide/L-lactide/δ-valerolactone
D,L-lactide/L-lactide/dioxanone
D,L-lactide/L-lactide

D,L-lactide/L-lactide/glycolide/ε-aprolactone
D,L-lactide/L-lactide/glycolide/TMC
D,L-lactide/L-lactide/glycolide/δ-valerolactone
D,L-lactide/L-lactide/glycolide/dioxanone
D,L-lactide/L-lactide/glycolide

Some examples of the resulting polyester gum base polymers include poly (L-lactide-co-trimethylenecarbonate); poly (L-lactide-co-epsilon-caprolactone); poly (D, L-lactide-co-trimethylenecarbonate); poly (D, L-lactide-co-epsilon-caprolactone); poly (meso-lactide-co-trimethylenecarbonate); poly (mesolactide-co-epsilon-caprolactone); poly (glycolide-cotrimethylenecarbonate); poly (glycolide-co-epsilon-caprolactone), etc. Suitable polyesters are also disclosed in WO 2004/028270, hereby incorporated by reference.

In an embodiment, the polyesters may be obtained by the reaction between at least one dimer acid and at least one glycol or alcohol. Such glycols can include, for example, glycerin, propylene glycol, ethylene glycol, poly(ethylene glycol), poly(propylene glycol), poly(ethylene glycol-co-propylene glycol), while such alcohols can include, for example, methanol, ethanol, propanol, and butanol, and such dimer acids can include, for example, adipic acid and citric acid, etc.

Some specific examples of suitable polyesters include poly(lactic acid), polylactide, poly(glycolic acid), polyglycolide, poly(citric acid), polycaprolactone, polyhydroxyalkanoate, and combinations thereof.

Some suitable prolamines include zein, corn gluten meal, wheat gluten, gliadin, glutenin and combinations thereof. Moreover, blends of prolamine with polyester such as those disclosed in US 6,858,238, hereby included by reference, may be useful in chewing gum according to the invention.

Agglomeration which may be used on e.g. tablet material and active ingredients in an embodiment of the invention may be performed for instance by fluid bed agglomeration, a process known to the person skilled in the art.

The chewing gum may include any component known in the chewing gum art. For example, the chewing gum may include elastomers, bulking agents, waxes, elastomer solvents, emulsifiers, plasticizers, fillers, and mixtures thereof.

The chewing gum according to the invention may comprise coloring agents. According to an embodiment of the invention, the chewing gum may comprise color agents and whiteners such as FD&C-type dyes and lakes, fruit and vegetable extracts, titanium dioxide and combinations thereof.

Further useful chewing gum base components include antioxidants, e.g. butylated hydroxytoluene (BHT), butyl hydroxyanisol (BHA), propylgallate and tocopherols, and preservatives.

A gum base formulation may, in accordance with the present invention, comprise one or more softening agents e.g. sucrose esters including those disclosed in WO 00/25598, which is incorporated herein by reference, tallow, hydrogenated tallow, hydrogenated and partially hydrogenated vegetable oils, cocoa butter, degreased cocoa powder, glycerol monostearate, glyceryl triacetate, lecithin, mono-, di- and triglycerides, acetylated monoglycerides, lanolin, sodium stearate, potassium stearate, glyceryl lecithin, propylene glycol monostearate, glycerine, fatty acids (e.g. stearic, palmitic, oleic and linoleic acids) and combinations thereof. As used herein the term "softener" designates an ingredient, which softens the gum base or chewing gum formulation and encompasses waxes, fats, oils, emulsifiers, surfactants and solubilisers.

To soften the gum base further and to provide it with water-binding properties, which confer to the gum base a pleasant smooth surface and reduce its adhesive properties, one or more emulsifiers is/are usually added to the composition, typically in an amount of 0 to 18% by weight, preferably 0 to 12% by weight of the gum base. Useful emulsifiers can include, but are not limited to, glyceryl monostearate, propylene glycol monostearate, mono- and diglycerides of edible fatty acids, lactic acid esters and acetic acid esters of mono- and diglycerides of edible fatty acids, acetylated mono and diglycerides, sugar esters of edible fatty acids, Na-, K-, Mg- and Ca-stearates, lecithin, hydroxylated lecithin and the like and mixtures thereof are examples of conventionally used emulsifiers which can be added to the chewing gum base. In case of the presence of a biologically or pharmaceutically active ingredient as defined below, the formulation may comprise certain specific emulsifiers and/or solubilisers in order to disperse and release the active ingredient.

Waxes and fats are conventionally used for the adjustment of the texture and for softening of the chewing gum base when preparing chewing gum bases. In connection with the present invention, any conventionally used and suitable type of natural and synthetic wax and fat may be used, such as for instance rice bran wax, polyethylene wax, petroleum wax (refined paraffin and microcrystalline wax), sorbitan monostearate, tallow, propylene glycol, paraffin, beeswax, carnauba wax, candelilla wax, cocoa butter, degreased cocoa powder and any suitable oil or fat, as e.g. completely or partially hydrogenated vegetable oils or completely or partially hydrogenated animal fats.

A chewing gum base formulation may, if desired, include one or more fillers/texturisers including as examples, magnesium and calcium carbonate, sodium sulphate, ground limestone, silicate compounds such as magnesium and aluminum silicate, kaolin and clay, aluminum oxide, silicium oxide, talc, titanium oxide, mono-, di- and tri-calcium phosphates, cellulose polymers, such as wood, and combinations thereof.

In addition to a water insoluble gum base portion, a typical chewing gum includes a water soluble bulk portion and one or more flavoring agents. The water-soluble portion may include bulk sweeteners, high-intensity sweeteners, flavoring agents, softeners, emulsifiers, colors, acidulants, buffering agents, fillers, antioxidants, and other components that provide desired attributes.

Combinations of sugar and/or non-sugar sweeteners can be used in the chewing gum formulation processed in accordance with the invention. Additionally, the softener may also provide additional sweetness such as aqueous sugar or alditol solutions.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

High-intensity artificial sweetening agents can also be used alone or in combination with the above sweeteners. Preferred high-intensity sweeteners include, but are not limited to sucralose, aspartame, salts of acesulfame, alitame, neotame, twinsweet, saccharin and its salts, cyclamic acid and its salts, glycyrrhizin, dihydrochalcones, thaumatin, monellin, stevioside and the like, alone or in combination. In order to provide longer lasting sweetness and flavor perception, it may be desirable to encapsulate or otherwise control the release of at least a portion of the artificial sweetener. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided using another chewing gum component such as a resinous compound.

Usage level of the high-intensity artificial sweetener will vary considerably and will depend on factors such as potency of the sweetener, rate of release, desired sweetness of the product, level and type of flavor used and cost considerations. Thus, the active level of high-potency artificial sweetener may vary from about 0 to about 8% by weight, preferably 0.001 to about 5% by weight. When carriers used for encapsulation are included, the usage level of the encapsulated sweetener will be proportionately higher.

If a low-calorie gum is desired, a low-caloric bulking agent can be used. Examples of low caloric bulking agents include polydextrose, Raftilose, Raftilin, fructooligosaccharides (NutraFlora^{®}), palatinose oligosaccharides; guar gum hydrolysates (e.g. Sun Fiber^{®}) or indigestible dextrins (e.g. Fibersol^{®}). However, other low-calorie bulking agents can be used.

The chewing gum according to the present invention may contain aroma agents and flavoring agents including natural and synthetic flavorings e.g. in the form of natural vegetable components, essential oils, essences, extracts, powders, including acids and other substances capable of affecting the taste profile. Examples of liquid and powdered flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

The chewing gum flavor may be a natural flavoring agent, which is freeze-dried, preferably in the form of a powder, slices or pieces or combinations thereof. The particle size may be less than 3 mm, less than 2 mm or more preferred less than 1 mm, calculated as the longest dimension of the particle. The natural flavoring agent may be in a form where the particle size is from about 3 µm to 2 mm, such as from 4 µm to 1 mm. Preferred natural flavoring agents include seeds from fruit e.g. from strawberry, blackberry and raspberry.

Various synthetic flavors, such as mixed fruit flavors may also be used in the present chewing gum centers. As indicated above, the aroma agent may be used in quantities smaller than those conventionally used. The aroma agents and/or flavors may be used in the amount from 0.01 to about 30% by weight of the final product depending on the desired intensity of the aroma and/or flavor used. Preferably, the content of aroma/flavor is in the range of 0.2 to 3% by weight of the total composition.

In an embodiment of the invention, the flavoring agents comprise natural and synthetic flavorings in the form of natural vegetable components, essential oils, essences, extracts, powders, including acids and other substances capable of affecting the taste profile.

In one embodiment of the invention, the flavor may be used as taste masking in chewing gum comprising active ingredients, which by themselves have undesired taste or which alter the taste of the formulation.

Further chewing gum ingredients, which may be included in the chewing gum according to the present invention, include surfactants and/or solubilisers, especially when pharmaceutically or biologically active ingredients are present. As examples of types of surfactants to be used as solubilisers in a chewing gum composition according to the invention, reference is made to H.P. Fiedler, Lexikon der Hilfstoffe für Pharmacie, Kosmetik und Angrenzende Gebiete, pages 63-64 (1981) and the lists of approved food emulsifiers of the individual countries. Anionic, cationic, amphoteric or non-ionic solubilisers can be used. Suitable solubilisers include lecithin, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllatylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds. In the presence of an active ingredient, the chewing gum may preferably also comprise a carrier known in the art.

Active ingredients may advantageously be applied in a chewing gum according to the invention. Active ingredients generally refer to those ingredients that are included in a delivery system and/or compressible chewing gum composition for the desired end benefit they provide to the user. In some embodiments, active ingredients can include medicaments, nutrients, nutraceuticals, herbals, nutritional supplements, pharmaceuticals, drugs, and the like and combinations thereof. Moreover, in the present context, active ingredients may refer to flavor components, high intensity sweeteners or other taste establishing components.

Active ingredients may be classified according to The Anatomical Therapeutic Chemical (ATC) classification system, which is a system for classification of medicinal products according to their primary constituent and to the organ or system on which they act and their chemical, pharmacological and therapeutic properties.

The first level of the ATC is split into 14 main groups based on the anatomical group:
A: Alimentary tract and metabolism
B: Blood and blood forming organs
C: Cardiovascular system
D: Dermatologicals
G: Genito urinary system and sex hormones
H: Systemic hormonal preparations, excl. sex hormones and insulins
J: Antiinfectives for systemic use
L: Antineoplastic and immunomodulating agents
M: Musculo-skeletal system
N: Nervous system
P: Antiparasitic products, insecticides and repellents
R: Respiratory system
S: Sensory organs
V: Various

Further subdivision is done into a second, third, fourth and fifth sub-group, which is based on the therapeutic main group, the therapeutic/pharmacological subgroup, the chemical/therapeutic/pharmacological subgroup, and the chemical substance subgroup respectively. In this sense each active ingredient has been given a unique ATC identification code indicating where the active ingredient may be useful.

However, as some active ingredients are useful in more than one area, some of the active ingredients mentioned in this document belong to two or more of the mentioned groups, e.g. phenylephrine, which has an ATC identification code in both C, R, and S, i.e. both C01CA06, R01AA04, R01AB01, R01BA03, S01FB01, and S01GA05 are ATC identification codes identifying phenylephrine.

The following list discloses examples of active ingredients which can be classified according to the ATC classification mentioned above and which are active ingredients which may be used in a chewing gum granule or a compressed chewing gum according to the invention:
Ephedrine, Magaldrate, Pseudoephedrine, Sildenafil, Xylocaine, Benzalconium chloride, Caffeine, Phenylephrine, Amfepramone, Orlistat, Sibutramine, Acetaminophen, Aspirin, Aluminum amino acetate, Aluminum amino acetate in combination with Magnesium oxide, Aluminum oxide hydrate in combination with Magnesiumoxide, Calcium carbonate in combination with Magnesium hydroxide, Calciumcarbonate, Dihydroxy Aluminum sodium carbonate, Magnesiumoxide, Glitazones, Metformin, Chlorpromazine, Dimenhydrinat, Domperidone, Meclozine, Metoclopramide, Odansetron, Prednisolone, Promethazine, Acrivastine, Cetirizine, Cinnarizine, Clemastine, Cyclizine, Desloratadine, Dexchlorpheniramine, Dimenhydrinate, Ebastine, Fexofenadine, Ibuprofen, Levolevoproricin, Loratadine, Meclozine, Mizolastine, Promethazine, Miconazole, Vitamin B12, Folic acid, Ferro compounds, vitamin C, Chlorhexidine diacetate, Fluoride, Decapeptide KSL, Aluminum fluoride, Aminochelated calcium, Ammonium fluoride, Ammonium fluorosilicate, Ammonium monofluorphosphate, Calcium fluoride, Calcium gluconate, Calcium glycerophosphate, Calcium lactate, Calcium monofluorphosphate, Calciumcarbonate, Carbamide, Cetyl pyridinium chloride, Chlorhexidine, Chlorhexidine digluconate, Chlorhexidine Chloride, Chlorhexidine diacetate, CPP Caseine Phospho Peptide, Hexetedine, Octadecentyl Ammonium fluoride, Potasium fluorosilicate, Potassium Chloride, Potassium monofluorphosphate, Sodium bi carbonate, Sodium carbonate, Sodium fluoride, Sodium fluorosilicate, Sodium monofluorphosphate, Sodium tri polyphosphate, Stannous fluoride, Stearyl Trihydroxyethyl Propylenediamine Dihydrofluoride, Strontium chloride, Tetra potassium pyrophosphate, Tetra sodium pyrophosphate, Tripotassium orthophosphate, Trisodium orthophosphate, Alginic acid, Aluminum hydroxide, Sodium bicarbonate, Sildenafil, Tadalafil, Vardenafil, Yohimbine, Cimetidine, Nizatidine, Ranitidine, Acetylsalicylic acid, Clopidogrel, Acetylcysteine, Bromhexine, Codeine, Dextromethorphan, Diphenhydramine, Noscapine, Phenylpropanolamine, vitamin D, Simvastatin, Bisacodyl, Lactitol, Lactulose, Magnesium oxide, Sodium picosulfate, Senna glycosides, Benzocaine, Lidocaine, Tetracaine, Almotriptan, Eletriptan, Naratriptan, Rizatriptan, Sumatriptan, Zolmitriptan, Calcium, Chromium, Copper, Iodine, Iron, Magnesium, Manganese, Molybdenium, Phosphor, Selenium, Zinc, Nicotine, Nicotine bitartrate, Nicotine pftalate, Nicotine polacrilex, Nicotine sulphate, Nicotine tartrate, Nicotine citrate, Nicotine lactate, Chloramine, Hydrogenperoxide, Metronidazole, Triamcinolonacetonide, Benzethonium Chl., Cetyl pyrid. Chl., Chlorhexidine, Fluoride, Lidocaine, Amphotericin, Miconazole, Nystatin, Fish oil, Ginkgo Biloba, Ginseng, Ginger, Purple coneflower, Saw Palmetto, Cetirizine, Levocetirizine, Loratadine, Diclofenac, Flurbiprofen, Acrivastine Pseudoephedrine, Loratadine Pseudoephedrine, Glucosamine, hyaluronic acid, Decapeptide KSL-W, Decapeptide KSL, Resveratrol, Misoprostol, Bupropion, Nicotine, Ondansetron HCl, Esomeprazole, Lansoprazole, Omeprazole, Pantoprazole, Rabeprazole, Bacteria and the like, Loperamide, Simethicone, Acetylsalicylic acid and others, Sucralfate, Vitamin A, Vitamin B1, Vitamin B12, Vitamin B2, Vitamin B6, Biotin, Vitamin C, Vitamin D, Vitamin E, Folinic acid, Vitamin K, Niacin, Q10, Clotrimazole, Fluconazole, Itraconazole, Ketoconazole, Terbinafine, Allopurinol, Probenecid, Atorvastatin, Fluvastatin, Lovastatin, Nicotinic acid, Pravastatin, Rosuvastatin, Simvastatin, Pilocarpine, Naproxen, Alendronate, Etidronate, Raloxifene, Risedronate, Benzodiazepines, Disulfiram, Naltrexone, Buprenorphine, Codeine, Dextropropoxyphene, Fentanyl, Hydromorphone, Ketobemidone, Ketoprofen, Methadone, Morphine, Naproxen, Nicomorphine, Oxycodone, Pethidine, Tramadol, Amoxicillin, Ampicillin, Azithromycin, Ciprofloxacin, Clarithromycin, Doxycyclin, Erythromycin, Fusidic acid, Lymecycline, Metronidazole, Moxifloxacin, Ofloxacin, Oxytetracycline, Phenoxymethylpenicillin, Rifamycins, Roxithromycin, Sulfamethizole, Tetracycline, Trimethoprim, Vancomycin, Acarbose, Glibenclamide, Gliclazide, Glimepiride, Glipizide, Insulin, Repaglinide, Tolbutamide, Oseltamivir, Aciclovir, Famciclovir, Penciclovir, Valganciclovir, Amlopidine, Diltiazem, Felodipine, Nifedipine, Verapamil, Finasteride, Minoxidil, Cocaine, Buphrenorphin, Clonidine, Methadone, Naltrexone, Calciumantagonists, Clonidine, Ergotamine, β-blockers, Aceclofenac, Celecoxib, Dexiprofen, Etodolac, Indometacin, Ketoprofen, Ketorolac, Lomoxicam, Meloxicam, Nabumetone, Oiroxicam, Parecoxib, Phenylbutazone, Piroxicam, Tiaprofenic acid, Tolfenamic acid, Aripiprazole, Chlorpromazine, Chlorprothixene, Clozapine, Flupentixol, Fluphenazine, Haloperidol, Lithium carbonate, Lithium citrate, Melperone, Penfluridol, Periciazine, Perphenazine, Pimozide, Pipamperone, Prochlorperazine, Risperidone, Thioridizin, Fluconazole, Itraconazole, Ketoconazole, Voriconazole, Opium, Benzodiazepines, Hydroxine, Meprobamate, Phenothiazine, Aluminiumaminoacetate, Esomeprazole, Famotidine, Magnesium oxide, Nizatide, Omeprazole, Pantoprazole, Fluconazole, Itraconazole, Ketoconazole, Metronidazole, Amphetamine, Atenolol, Bisoprolol fumarate, Metoprolol, Metropolol, Pindolol, Propranolol, Auranofm, and Bendazac.

Further examples of useful active ingredients include active ingredients selected from the therapeutical groups comprising: Analgesic, Anaestetic, Antipyretic, Anti allergic, Anti-arrytmic, Appetite suppressant, Antifungal, Anti-inflammatory, Broncho dilator, Cardiovascular drugs, Coronary dilator, Cerebral dilator, Peripheral vasodilator, Anti-infective, Psychotropic, Anti- manic, Stimulant, Antihistamine, Laxative, Decongestrant, Gastro-intestinal sedative, Sexual dysfunction agent, Desinfectants, Anti-diarrheal, Anti-anginal substance, Vasodilator, Anti-hypertensive agent, Vasoconstrictor, Migraine treating agent, Antibiotic, Tranquilizer, Ntipsychotic, Anti-tumor drug, Anticoagulant, Antithrombotic agent, Hypnotic, Sedative, Anti-emetic, Anti-, auseant, Anticonvulsant, Neuromuscular agent, Hyper and hypoglycaemic, Thyroid and antithyroid, Diuretic, Antispasmodic, Uterine relaxant, Anti-obesity agent, Anoretic, Spasnolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretc, Anti-flatulent, Betablokker, Teeth Whitener, Enzyme, Co-enzyme, Protein, Energy booster, Fiber, Probiotics, Prebiotics, Antimicrobial agent, NSAID, Anti-tussives, Decongestrants, Anti-histamines, Expectorants, Anti-diarrheals, Hydrogen antagonists, Proton pump inhibitors, General nonselective CNS depressants, General nonselective CNS stimulants, Selectively CNS function modyfying drugs, Antiparkinsonism, Narcotic-analgetics, Analgetic-antipyretics, Psychopharmacological drugs, and Sexual dysfunction agents.

Examples of useful active ingredients include: Casein glyco-macro-peptide (CGMP), Nicotine, Nicotine bitartrate, Nicotine sulphate, Nicotine tartrate, Nicotine pftalate, Nicotine lactate, Nicotinecitrate, Nicotine polacrilex, Triclosan, Cetyl pyridinium chloride, Domiphen bromide, Quartemary ammonium salts, Zinc components, Sanguinarine, Fluorides, Alexidine, Octonidine, EDTA, Aspirin, Acetaminophen, Ibuprofen, Ketoprofen, Diflunisal, Fenoprofen calcium, Naproxen, Tolmetin sodium, Indomethacin, Benzonatate, Caramiphen edisylate, Menthol, Dextromethorphan hydrobromide, Theobromine hydrochloride, Chlophendianol Hydrochloride, Pseudoephedrine Hydrochloride, Phenylephrine, Phenylpropanolamine, Pseudoephedrine sulphate, Brompheniramine maleate, Chlorpheniramine- maleate, Carbinoxamine maleate, Clemastine fumarate, Dexchlorpheniramine maleate, Dephenhydramine hydrochloride, Diphenpyralide hydrochloride, Azatadine maleate, Diphenhydramine citrate, Doxylamine succinate, Promethazine hydrochloride, Pyrilamine maleate, Tripellenamine citrate, Triprolidine hydrochloride, Acrivastine, Loratadine, Brompheniramine, Dexbrompheniamine, Guaifenesin, Ipecac, Potassium iodide, Terpin hydrate, Loperamide, Famotidine, Ranitidine, Omeprazole, Lansoprazole, Aliphatic alcohols, Barbiturates, Caffeine, Nicotine, Strychnine, Picrotoxin, Pentyenetetrazol, Phenyhydantoin, Phenobarbital, Primidone, Carbamazapine, Etoxsuximide, Methsuximide, Phensuximide, Trimethadione, Diazepam, Benzodiazepines, Phenacemide, Pheneturide, Acetazolamide, Sulthiame, Bromide, Levodopa, Amantadine, Morphine, Heroin, Hydromorphone, Metopon, Oxymorphone, Levophanol, Codeine, Hydrocodone, Xycodone, Nalorphine, Naloxone, Naltrexone, Salicylates, Phenylbutazone, Indomethacin, Phenacetin, Chlorpromazine, Methotrimeprazine, Haloperidol, Clozapine, Reserpine, Imipramine, Tranylcypromine, Phenelzine, Lithium, Sildenafil citrate, Tadalafil, and Vardenafil CL.

Examples of useful active ingredients include active ingredients selected from the groups of ace-inhibitors, antianginal drugs, anti- arrhythmias, anti-asthmatics, anti-cholesterolemics, analgesics, anesthetics, anticonvulsants, anti-depressants, antidiabetic agents, anti-diarrhea preparations, antidotes, anti-histamines, anti-hypertensive drugs, anti-inflammatory agents, anti-lipid agents, anti- manics, anti-nauseants, anti-stroke agents, anti-thyroid preparations, anti-tumor drugs, anti- viral agents, acne drugs, alkaloids, amino acid preparations, anti-tussives, anti- uricemic drugs, anti- viral drugs, anabolic preparations, systemic and non-systemic anti-infective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocriptine or nicotine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of active ingredients contemplated for use in the present invention can include antacids, H2-antagonists, and analgesics. For example, antacid dosages can be prepared using the ingredients calcium carbonate alone or in combination with magnesium hydroxide, and/or aluminum hydroxide. Moreover, antacids can be used in combination with H2-antagonists.

Analgesics include opiates and opiate derivatives, such as Oxycontin™, ibuprofen, aspirin, acetaminophen, and combinations thereof that may optionally include caffeine.

Other drug active ingredients for use in embodiments can include anti-diarrheals such as Immodium™ AD, anti-histamines, anti-tussives, decongestants, vitamins, and breath fresheners. Also contemplated for use herein are anxiolytics such as Xanax™; anti-psychotics such as Clozaril™ and Haldol™; non-steroidal antiinflammatories (NSAID's) such as ibuprofen, naproxen sodium, Voltaren™ and Lodine™, anti-histamines such as Claritin™, Hismanal™, Relafen™ and Tavist™; antiemetics such as Kytril™ and Cesamet™; bronchodilators such as Bentolin™, Proventil™; anti-depressants such as Prozac™, Zoloft™, and Paxil™; anti-migraines such as Imigra™, ACE-inhibitors such as Vasotec™, Capoten™ and Zestril™; anti-Alzheimer's agents, such as Nicergoline™; and CaH-antagonists such as Procardia™, Adalat™, and Calan™.

The popular H2-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients can include, but are not limited to, the following: aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts. A variety of nutritional supplements may also be used as active ingredients including virtually any vitamin or mineral. For example, vitamin A, vitamin C, vitamin D, vitamin E, vitamin K, vitamin B6, vitamin B12, thiamine, riboflavin, biotin, folic acid, niacin, pantothenic acid, sodium, potassium, calcium, magnesium, phosphorus, sulfur, chlorine, iron, copper, iodine, zinc, selenium, manganese, choline, chromium, molybdenum, fluorine, cobalt and combinations thereof, may be used. Examples of nutritional supplements that can be used as active ingredients are set forth in U.S. Patent Application Publication Nos. 2003/0157213 Al, 2003/0206993 and 2003/0099741 Al which are incorporated in their entirety herein by reference for all purposes. Various herbals may also be used as active ingredients such as those with various medicinal or dietary supplement properties. Herbals are generally aromatic plants or plant parts and or extracts thereof that can be used medicinally or for flavoring. Suitable herbals can be used singly or in various mixtures. Commonly used herbs include Echinacea, Goldenseal, Calendula, Rosemary, Thyme, Kava Kava, Aloe, Blood Root, Grapefruit Seed Extract, Black Cohosh, Ginseng, Guarana, Cranberry, Ginko Biloba, St. John's Wort, Evening Primrose Oil, Yohimbe Bark, Green Tea, Ma Huang, Maca, Bilberry, Lutein, and combinations thereof.

Especially when hydrophilic, encapsulation of the active will result in a delay in the release of the predominant amount of the active during consumption of a compressible chewing gum that includes the encapsulated active (e.g., as part of a delivery system added as an ingredient to the compressible chewing gum), in some embodiments, the release profile of the ingredient (e.g., the active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more components of an effervescing system are managed for a compressible gum. The effervescent system may include one or more edible acids and one or more edible alkaline materials. The edible acid(s) and the edible alkaline material(s) may react together to generate effervescence. In some embodiments, the alkaline material(s) may be selected from, but is not limited to, alkali metal carbonates, alkali metal bicarbonates, alkaline earth metal carbonates, alkaline earth metal bicarbonates, and combinations thereof. The edible acid(s) may be selected from, but is not limited to, citric acid, phosphoric acid, tartaric acid, malic acid, ascorbic acid, and combinations thereof. In some embodiments, an effervescing system may include one or more other ingredients such as, for example, carbon dioxide, oral care ingredients, flavorants, etc.

For examples of use of an effervescing system in a chewing gum, refer to U.S. Provisional Patent No. 60/618,222 filed October 13, 2004, and entitled "Effervescent Pressed Gum Tablet Compositions," the contents of which are incorporated herein by reference for all purposes. Other examples can be found in U.S. Patent No. 6,235,318, the contents of which are incorporated herein by reference for all purposes. Typically, encapsulation of the one or more ingredients in an effervescing system will result in a delay in the release of the predominant amount of the one or more ingredients during consumption of a compressible chewing gum that includes the encapsulated one or more ingredients (e.g., as part of a delivery system added as an ingredient to the compressible chewing gum composition). The release profile of the one or more ingredients can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more appetite suppressors are managed for a compressible gum. Appetite suppressors can be ingredients such as fiber and protein that function to depress the desire to consume food. Appetite suppressors can also include benzphetamine, diethylpropion, mazindol, phendimetrazine, phentermine, hoodia (P57), Olibra™, ephedra, caffeine and combinations thereof. Appetite suppressors are also known by the following trade names: Adipex™, Adipost™, Bontril™ PDM, Bontril™ Slow Release, Didrex™, Fastin™, Ionamin™, Mazanor™, Melfiat™, Obenix™, Phendiet™, Phendiet-105™, Phentercot™, Phentride™, Plegine™, Prelu-2™, Pro-Fast™, PT 105™, Sanorex™, Tenuate™, Sanorex™, Tenuate™, Tenuate Dospan™, Tepanil Ten-Tab™, Teramine™, and Zantryl™. These and other suitable appetite suppressors are further described in the following U.S. patents, all of which are incorporated in their entirety by reference hereto: U.S. 6,838,431 to Portman, U.S. 6,716,815 to Portman, U.S. 6,558,690 to Portman, U.S. 6,468,962 to Portman, U.S. 6,436,899 to Portman.

Typically, encapsulation of the appetite suppressor will result in a delay in the release of the predominant amount of the appetite suppressor during consumption of a compressible chewing gum that includes the encapsulated appetite suppressor (e.g., as part of a delivery system added as an ingredient to the compressible chewing gum). In some embodiments, the release profile of the ingredient (e.g., the appetite suppressor) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more breath fresheners are managed for a compressible gum. Breath fresheners can include essential oils as well as various aldehydes, alcohols, and similar materials. In some embodiments, essential oils can include oils of spearmint, peppermint, wintergreen, sassafras, chlorophyll, citral, geraniol, cardamom, clove, sage, carvacrol, eucalyptus, cardamom, magnolia bark extract, marjoram, cinnamon, lemon, lime, grapefruit, and orange. In some embodiments, aldehydes such as cinnamic aldehyde and salicylaldehyde can be used. Additionally, chemicals such as menthol, carvone, iso-garrigol, and anethole can function as breath fresheners. Of these, the most commonly employed are oils of peppermint, spearmint and chlorophyll.

In addition to essential oils and chemicals derived from them, in some embodiments, breath fresheners can include but are not limited to zinc citrate, zinc acetate, zinc fluoride, zinc ammonium sulfate, zinc bromide, zinc iodide, zinc chloride, zinc nitrate, zinc flurosilicate, zinc gluconate, zinc tartarate, zinc succinate, zinc formate, zinc chromate, zinc phenol sulfonate, zinc dithionate, zinc sulfate, siliver nitrate, zinc salicylate, zinc glycerophosphate, copper nitrate, chlorophyll, copper chlorophyll, chlorophyllin, hydrogenated cottonseed oil, chlorine dioxide, beta cyclodextrin, zeolite, silica-based materials, carbon-based materials, enzymes such as laccase, and combinations thereof. In some embodiments, the release profiles of probiotics can be managed for a compressible gum including, but not limited to lactic acid producing microorganisms such as Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides, Bacillus coagulans, Bacillus subtilis, Bacillus laterosporus, Bacillus laevolacticus, Sporolactobacillus inulinus and mixtures thereof. Breath fresheners are also known by the following trade names: Retsyn™, Actizol™, and Nutrazin™. Examples of malodor-controlling compositions are also included in U.S. Patent No. 5,300,305 to Stapler et al. and in U.S. Patent Application Publication Nos. 2003/0215417 and 2004/0081713 which are incorporated in their entirety herein by reference for all purposes.

Typically, encapsulation of the breath-freshening ingredient will result in a delay in the release of the predominant amount of the active during consumption of a compressible chewing gum that includes the encapsulated breath-freshening ingredient (e.g., as part of a delivery system added as an ingredient to the compressible chewing gum composition). In some embodiments, the release profile of the ingredient (e.g., the breath-freshening ingredient) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more dental care ingredients may be managed for a compressible gum. Such dental care ingredients (also known as oral care ingredients) may include but are not limited to tooth whiteners, stain removers, oral cleaning, bleaching agents, desensitizing agents, dental remineralization agents, antibacterial agents, anticaries agents, plaque acid buffering agents, surfactants and anticalculus agents. Non-limiting examples of such ingredients can include, hydrolytic agents including proteolytic enzymes, abrasives such as hydrated silica, calcium carbonate, sodium bicarbonate and alumina, other active stain-removing components such as surface-active agents, including, but not limited to anionic surfactants such as sodium stearate, sodium palminate, sulfated butyl oleate, sodium oleate, salts of fumaric acid, glycerol, hydroxylated lecithin, sodium lauryl sulfate and chelators such as polyphosphates, which are typically employed as tartar control ingredients. In some embodiments, dental care ingredients can also include tetrasodium pyrophosphate and sodium tri-polyphosphate, sodium bicarbonate, sodium acid pyrophosphate, sodium tripolyphosphate, xylitol, sodium hexametaphosphate. In some embodiments, peroxides such as carbamide peroxide, calcium peroxide, magnesium peroxide, sodium peroxide, hydrogen peroxide, and peroxydiphospate are included. In some embodiments, potassium nitrate and potassium citrate are included. Other examples can include casein glycomacropeptide, calcium casein peptone-calcium phosphate, casein phosphopeptides, casein phosphopeptide- amorphous calcium phosphate (CPP-ACP), and amorphous calcium phosphate. Still other examples can include papaine, krillase, pepsin, trypsin, lysozyme, dextranase, mutanase, glycoamylase, amylase, glucose oxidase, and combinations thereof. Further examples can include surfactants such as sodium stearate, sodium ricinoleate, and sodium lauryl sulfate surfactants for use in some embodiments to achieve increased prophylactic action and to render the dental care ingredients more cosmetically acceptable. Surfactants can preferably be detersive materials which impart to the composition detersive and foaming properties. Suitable examples of surfactants are water-soluble salts of higher fatty acid monoglyceride monosulfates, such as the sodium salt of the monosulfated monoglyceride of hydgrogenated coconut oil fatty acids, higher alkyl sulfates such as sodium lauryl sulfate, alkyl aryl sulfonates such as sodium dodecyl benzene sulfonate, higher alkyl sulfoacetates, sodium lauryl sulfoacetate, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine. In addition to surfactants, dental care ingredients can include antibacterial agents such as, but not limited.to, triclosan, chlorhexidine, zinc citrate, silver nitrate, copper, limonene, and cetyl pyridinium chloride. In some embodiments, additional anticaries agents can include fluoride ions or fluorine-providing components such as inorganic fluoride salts. In some embodiments, soluble alkali metal salts, for example, sodium fluoride, potassium fluoride, sodium fluorosilicate, ammonium fluorosilicate, sodium monofluorophosphate, as well as tin fluorides, such as stannous fluoride and stannous chloride can be included. In some embodiments, a fluorine-containing compound having a beneficial effect on the care and hygiene of the oral cavity, e.g., diminution of enamel solubility in acid and protection of the teeth against decay may also be included as an ingredient. Examples thereof include sodium fluoride, stannous fluoride, potassium fluoride, potassium stannous fluoride (SnF.sub.2 -KF), sodium hexafluorostannate, stannous chlorofluoride, sodium fluorozirconate, and sodium monofluorophosphate. In some embodiments, urea is included. Further examples are included in the following U.S. patents and U.S. published patent applications, the contents of all of which are incorporated in their entirety herein by reference for all purposes: U.S. Patent Nos. 5,227,154 to Reynolds, 5,378,131 to Greenberg, 6,846,500 to Luo et al, 6,733,818 to Luo et al., 6,696,044 to Luo et al., 6,685,916 to Holme et al., 6,485,739 to Luo et al., 6,479,071 to Holme et al., 6,471,945 to Luo et al., U.S. Patent Publication Nos. 20050025721. to Holme et al., 2005008732 to Gebreselassie et al., and 20040136928 to Holme et al.

Typically, encapsulation of the active will result in a delay in the release of the predominant amount of the active during consumption of a compressible chewing gum that includes the encapsulated active (e.g., as part of a delivery system added as an ingredient to the compressible chewing gum composition). In some embodiments, the release profile of the ingredient (e.g., the dental care active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more flavor potentiators can be managed for a compressible gum. Flavor potentiators can consist of materials that may intensify, supplement, modify or enhance the taste and/or aroma perception of an original material without introducing a characteristic taste and/or aroma perception of their own. In some embodiments, potentiators designed to intensify, supplement, modify, or enhance the perception of flavor, sweetness, tartness, umami, kokumi, saltiness and combinations thereof can be included. In some embodiments, sweetness may be potentiated by the inclusion of monoammonium glycyrrhizinate, licorice glycyrrhizinates, citrus aurantium, maltol, ethyl maltol, vanilla, vanillin, and combinations thereof. In some embodiments, sugar acids, sodium chloride, potassium chloride, sodium acid sulfate, and combinations thereof may be included for flavor potentiation. In other examples, glutamates such as monosodium glutamate (MSG), monopotassium glutamate, hydrolyzed vegetable protein, hydrolyzed animal protein, yeast extract, and combinations thereof are included. Further examples can include glutathione, and nucleotides such as inosine monophosphate (IMP), disodium inosinate, xanthosine monophosphate, guanylate monophosphate (GMP), and combinations thereof. For bitterness blocking or taste masking, ingredients that interact with bitterness receptors to suppress bitterness or off tastes may be included. In some embodiments, adenosine monophosphate (AMP) can be included for bitterness suppression. Bitterness modification can also be accomplished by using sweetness or flavors with complementary bitter notes such as chocolate. Further examples of flavor potentiator compositions that impart kokumi are also included in U.S. Patent No. 5,679,397 to Kuroda et al, the entire contents of which are incorporated in its entirety herein by reference.

Typically, encapsulation of a flavor potentiator will result in a delay in the release of the predominant amount of the flavor potentiator during consumption of a compressible chewing gum that includes the encapsulated flavor potentiator (e.g., as part of a delivery system added as an ingredient to the compressible chewing gum composition). In some embodiments, the release profile of the ingredient (e.g., the flavor potentiator) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more acids may be managed for a compressible gum. Acids can include, but are not limited to acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof.

Typically, encapsulation of a food acid will result in a delay in the release of the predominant amount of the active during consumption of a compressible chewing gum that includes the encapsulated food acid (e.g., as part of a delivery system added as an ingredient to the compressible chewing gum). In some embodiments, the release profile of the ingredient (e.g., the food acid) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more micronutrients can be managed for a compressible gum. Micronutrients can include materials that have an impact on the nutritional wellbeing of an organism even though the quantity required by the organism to have the desired effect is small relative to macronutrients such as protein, carbohydrate, and fat. Micronutrients can include, but are not limited to vitamins, minerals, enzymes, phytochemicals, antioxidants, and combinations thereof. In some embodiments, vitamins can include fat soluble vitamins such as vitamin A, vitamin D, vitamin E, and vitamin K and combinations thereof, in some embodiments, vitamins can include water soluble vitamins such as vitamin C (ascorbic acid), the B vitamins (thiamine or B1, riboflavoin or B2, niacin or B3, pyridoxine or B6, folic acid or B9, cyanocobalimin or B12, pantothenic acid, biotin), and combinations thereof.

In some embodiments, minerals can include but are not limited to sodium, magnesium, chromium, iodine, iron, manganese, calcium, copper, fluoride, potassium, phosphorous, molybdenum, selenium, zinc, and combinations thereof.

In some embodiments micronutrients can include but are not limited to L- carnitine, choline, coenzyme Q10, alpha-lipoic acid, omega-3 -fatty acids, pepsin, phytase, trypsin, lipases, proteases, cellulases, and combinations thereof.

Antioxidants can include materials that scavenge free radicals. In some embodiments, antioxidants can include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In some embodiments, phytochemicals can include but are not limited to cartotenoids, chlorophyll, chlorophyllin, fiber, flavanoids, anthocyanins, cyaniding, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof.

Typically, encapsulation of the micronutrient will result in a delay in the release of the predominant amount of the active during consumption of a compressible chewing gum that includes the encapsulated micronutrient (e.g., as part of a delivery system added as an ingredient to the compressible chewing gum). In some embodiments, the release profile of the ingredient (e.g., the micronutrient) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more mouth moisteners can be managed for a compressible gum. Mouth moisteners can include, but are not limited to, saliva stimulators such as acids and salts and combinations thereof. In some embodiments, acids can include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof. Mouth moisteners can also include hydrocolloid materials that hydrate and may adhere to oral surface to provide a sensation of mouth moistening. Hydrocolloid materials can include naturally occurring materials such as plant exudates, seed gums, and seaweed extracts or they can be chemically modified materials such as cellulose, starch, or natural gum derivatives. In some embodiments, hydrocolloid materials can include pectin, gum arabic, acacia gum, alginates, agar, carageenans, guar gum, xanthan gum, locust bean gum, gelatin, gellan gum, galactomannans, tragacanth gum, karaya gum, curdlan, konjac, chitosan, xyloglucan, beta glucan, furcellaran, gum ghatti, tamarin, bacterial gums, and combinations thereof. Additionally, in some embodiments, modified natural gums such as propylene glycol alginate, carboxymethyl locust bean gum, low methoxyl pectin, and their combinations can be included. In some embodiments, modified celluloses can be included such as microcrystalline cellulose, carboxymethlcellulose (CMC), methylcellulose (MC), hydroxypropylniethylcellulose (HPCM), and hydroxypropylcellulose (MPC), and combinations thereof. Similarly, humectants which can provide a perception of mouth hydration can be included. Such humectants can include, but are not limited to glycerol, sorbitol, polyethylene glycol, erythritol, and xylitol. Additionally, in some embodiments, fats can provide a perception of mouth moistening. Such fats can include medium chain triglycerides, vegetable oils, fish oils, mineral oils, and combinations thereof. Typically, encapsulation of a mouth moistening agent will result in a delay in the release of the predominant amount of the active during consumption of a compressible chewing gum that includes the encapsulated mouth moistening agent (e.g., as part of a delivery system added as an ingredient to the compressible chewing gum). In some embodiments, the release profile of the ingredient (e.g., the mouth moistening agent) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more ingredients that soothe the throat can be managed for a compressible gum. Throat soothing ingredients can include analgesics, anesthetics, demulcents, antiseptic, and combinations thereof. In some embodiments, analgesics/anesthetics can include menthol, phenol, hexylresorcinol, benzocaine, dyclonine hydrochloride, benzyl alcohol, salicyl alcohol, and combinations thereof. In some embodiments, demulcents can include but are not limited to slippery elm bark, pectin, gelatin, and combinations thereof. In some embodiments, antiseptic ingredients can include cetylpyridinium chloride, domiphen bromide, dequalinium chloride, and combinations thereof.

In some embodiments, antitussive ingredients such as chlophedianol hydrochloride, codeine, codeine phosphate, codeine sulfate, dextromethorphan, dextromethorphan hydrobromide, diphenhydramine citrate, and diphenhydramine hydrochloride, and combinations thereof can be included.

In some embodiments, throat soothing agents such as honey, propolis, aloe vera, glycerine, menthol and combinations thereof can be included. In still other embodiments, cough suppressants can be included. Such cough suppressants can fall into two groups: those that alter the texture or production of phlegm such as mucolytics and expectorants; and those that suppress the coughing reflex such as codeine (narcotic cough suppressants), antihistamines, dextromethorphan and isoproterenol (non-narcotic cough suppressants). In some embodiments, ingredients from either or both groups can be included.

In still other embodiments, antitussives can include, but are not limited to, the group consisting of codeine, dextromethorphan, dextrorphan, diphenhydramine, hydrocodone, noscapine, oxycodone, pentoxyverine and combinations thereof. In some embodiments, antihistamines can include, but are not limited to, acrivastine, azatadine, brompheniramine, chlo[phi]heniramine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof. In some embodiments, non-sedating antihistamines can include, but are not limited to, astemizole, cetirizine, ebastine, fexofenadine, loratidine, terfenadine, and combinations thereof.

In some embodiments, expectorants can include, but are not limited to, ammonium chloride, guaifenesin, ipecac fluid extract, potassium iodide and combinations thereof. In some embodiments, mucolytics can include, but are not limited to, acetylcycsteine, ambroxol, bromhexine and combinations thereof. In some embodiments, analgesic, antipyretic and anti-inflammatory agents can include, but are not limited to, acetaminophen, aspirin, diclofenac, diflunisal, etodolac, fenoprofen, flurbiprofen, ibuprofen, ketoprofen, ketorolac, nabumetone, naproxen, piroxicam, caffeine and mixtures thereof. In some embodiments, local anesthetics can include, but are not limited to, lidocaine, benzocaine, phenol, dyclonine, benzonotate and mixtures thereof. In some embodiments nasal decongestants and ingredients that provide the perception of nasal clearing can be included. In some embodiments, nasal decongestants can include but are not limited to phenylpropanolamine, pseudoephedrine, ephedrine, phenylephrine, oxymetazoline, and combinations thereof. In some embodiments ingredients that provide a perception of nasal clearing can include but are not limited to menthol, camphor, borneol, ephedrine, eucalyptus oil, peppermint oil, methyl salicylate, bornyl acetate, lavender oil, wasabi extracts, horseradish extracts, and combinations thereof. In some embodiments, a perception of nasal clearing can be provided by odoriferous essential oils, extracts from woods, gums, flowers and other botanicals, resins, animal secretions, and synthetic aromatic materials.

Typically, encapsulation of a throat care agent will result in a delay in the release of the predominant amount of the active during consumption of a compressible chewing gum that includes the encapsulated throat care agent (e.g. as part of a delivery system added as an ingredient to the compressible chewing gum). In some embodiments, the release profile of the ingredient (e.g. the dental care active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof. In some embodiments, exempt from certification or natural colors can include, but are not limited to annatto extract, (E 160b), bixin, norbixin, astaxanthin, dehydrated beets (beet powder), beetroot red/betanin (E 162), ultramarine blue, canthaxanthin (E 161 g), cryptoxanthin (E 161 c), rubixanthin (E161d), violanxanthin (E161e), rhodoxanthin (E161f), caramel (E150(a-d)), β-apo-8'-carotenal (E160e), β-carotene (E160a), alpha carotene, gamma carotene, ethyl ester of beta-apo-8 carotenal (E160f), fiavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some embodiments, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes. These consist of the aluminum salts of FD&C dyes extended on an insoluble substrate of alumina hydrate. Additionally, in some embodiments, certified colors can be included as calcium salts. Typically, encapsulation of a color will result in a delay in the release of the predominant amount of the active during consumption of a compressible chewing gum that includes the encapsulated color (e.g., as part of a delivery system added as an ingredient to the compressible chewing gum). In some embodiments, the release profile of the ingredient (e.g., the color) can be managed by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, a delivery system or compressible chewing gum may include two or more ingredients for which managed release from the compressible chewing gum during consumption of the compressible chewing gum is desired. In some embodiments, the ingredients may be encapsulated or otherwise included separately in different delivery systems. Alternatively, in some embodiments the ingredients may be encapsulated or otherwise included in the same delivery system. As another possibility, one or more of the ingredients may be free (e.g. unencapsulated) while one or more other ingredients may be encapsulated. A compressible chewing gum may include a group of ingredients for which managed release of the group during consumption of the compressible chewing gum is desired. Groups of two or more ingredients for which managed release from a compressible chewing gum during consumption of the compressible chewing gum may be desired include, but are not limited to: color and flavor, multiple flavors, multiple colors, cooling agent and flavor, warming agent and flavor, cooling agent and warming agent, cooling agent and high-intensity sweetener, warming agent and high-intensity sweetener, multiple cooling agents (e.g., WS-3 and WS-23, WS-3 and menthyl succinate), menthol and one or more cooling agents, menthol and one or more warming agents, multiple warming agents, high-intensity sweetener(s) and tooth whitening active(s), high-intensity sweetener(s) and breath-freshening active(s), an ingredient with some bitterness and a bitterness suppressor for the ingredient, multiple high-intensity sweeteners (e.g., ace-k and aspartame), multiple tooth whitening actives (e.g., an abrasive ingredient and an antimicrobial ingredient, a peroxide and a nitrate, a warming agent and a polyol, a cooling agent and a polyol, multiple polyols, a warming agent and micronutrient, a cooling agent and a micronutrient, a warming agent and a mouth moistening agent, a cooling agent and a mouth moistening agent, a warming agent and a throat care agent, a cooling agent and a throat care agent, a warming agent and a food acid, a cooling agent and food acid, a warming agent and an emulsifier/surfactant, a cooling agent and an emulsifier/surfactant, a warming agent and a color, a cooling agent and a color, a warming agent and a flavor potentiator, a cooling agent and a flavor potentiator, a warming agent with sweetness potentiator, a cooling agent with a sweetness potentiator, a warming agent and an appetite suppressant, a cooling agent and an appetite suppressant, a high-intensity sweetener and a flavor, a cooling agent and a teeth-whitening agent, a warming agent and a teeth-whitening agent, a warming agent and breath-freshening agent, a cooling agent and a breath-freshening agent, a cooling agent and an effervescing system, a warming agent and an effervescing system, a warming agent and an antimicrobial agent, a cooling agent and an antimicrobial agent, multiple anticalcums ingredients, multiple remineralization ingredients, multiple surfactants, remineralization ingredients with demineralization ingredients, acidic ingredients with acid buffering ingredients, anticalculus ingredients with antibacterial ingredients, remineralization ingredients with anticalculus ingredients, anticalculus ingredients with remineralization ingredients with antibacterial ingredients, surfactant ingredients with anticalculus ingredients, surfactant ingredients with antibacterial ingredients, surfactant ingredients with remineralization ingredients, surfactants with anticalculus ingredients with antibacterial ingredients, multiple types of vitamins or minerals, multiple micronutrients, multiple acids, multiple antimicrobial ingredients, multiple breath-freshening ingredients, breath-freshening ingredients and antimicrobial ingredients, multiple appetite suppressors, acids and bases that react to effervesce, a bitter compound with a high-intensity sweetener, a cooling agent and an appetite suppressant, a warming agent and an appetite suppressant, a high-intensity sweetener and an appetite suppressant, a high-intensity sweetener with an acid, a probiotic ingredient and a prebiotic ingredient, a vitamin and a mineral, a metabolic enhancement ingredient with a macronutrient, a metabolic enhancement ingredient with a micronutrient, an enzyme with a substrate, a high-intensity sweetener with a sweetness potentiator, a cooling compound with a cooling potentiator, a flavor with a flavor potentiator, a warming compound with a warming potentiator, a flavor with salt, a high-intensity sweetener with salt, an acid with salt, a cooling compound with salt, a warming compound with salt, a flavor with a surfactant, an astringent compound with an ingredient to provide a sensation of hydration, etc. In some embodiments, the multiple ingredients may be part of the same delivery system or may be part of different delivery systems. Different delivery systems may use the same or different encapsulating materials.

Typically, encapsulation of the multiple ingredients will result in a delay in the release of the predominant amount of the multiple ingredients during consumption of a compressible chewing gum that includes the encapsulated multiple ingredients (e.g. as part of a delivery system added as an ingredient to the compressible chewing gum). This may be particularly helpful in situations wherein separate encapsulation of the ingredients may cause them to release with different release profiles. For example, different high-intensity sweeteners may have different release profiles because they have different water solubilities or differences in other characteristics. Encapsulating them together may cause them to release more simultaneously.

In some embodiments, the release profile of the multiple ingredients can be managed for a compressible gum by managing various characteristics of the multiple ingredients, the delivery system containing the multiple ingredients, and/or the compressible chewing gum containing the delivery system and/or how the delivery system is made in a manner as previously discussed above.

The active ingredients mentioned above are meant as examples of active ingredients which could be applicable in a chewing gum granule or compressed chewing gum, however, this list should not be considered as exhaustive.

Active ingredients to be applied in tablets according to embodiments of the invention may be applied as such or be included or bonded in different ways, such as being part of an inclusion complex e.g. as described in US 5,866,179, which is hereby incorporated by reference. A resin-bonding of nicotine is described in e.g. WO 2006/000232 which is also incorporated herein by reference. Further conventional methods of applying active ingredients may obviously be applied within the scope of the invention.

The active ingredients may advantageously be applied in a gum base-containing module or a tablet-module substantially free of gum base depending on the applied type of active ingredient. If the active ingredient is of the pharmaceutical type, such ingredient may very often advantageously be comprised in a tablet module substantially free of gum base whereas taste relevant active ingredients advantageously may be added to the gum base-containing module and very often to both types of modules. The taste relevant active ingredient may both be added as separate particles which are mixed and compressed with gum base-containing particles in one module and it may be incorporated into gum base-containing granules.

In the present context, the terms granule and particle are used interchangeable in the sense that a granule or particle for use in a compression process is regarded to be a relatively small object, which together with other granules or particles may be compressed into a stable chewing gum tablet. The granules or particles may be produced in several different ways. A gum base-containing granule of particle may typically be produced substantially into the desired shape by means of an extrusion process or alternatively be produced on the basis of a gum base-containing mass which is subsequently separated into particles of a smaller size.

The following non-limiting examples illustrate the manufacturing of granules, chewing gums, and the use of different free-flowing agents including the evaluation of these.

### Example 1

### Preparation of gum base

The applied gum base had the following composition and was prepared as gum base pellets in a conventional mixing process.

| | |
|---|---|
| elastomer: | 19 % by weight |
| natural resin: | 20% by weight |
| synthetic resin: | 20 % by weight |
| fat/fillers: | 26 % by weight |
| wax: | 15 % by weight |

Obviously within the scope of the invention gum base may be prepared by other processes such as a one-step process or any other conventional process.

### Example 2

### Preparation of chewing gum granules

The gum base of example 1 was used in the manufacture of chewing gum products according to embodiments of the invention.

An extruder (Leistritz ZSE/BL 360 kw 104, available from Leistritz GmbH, Germany) extruded the composition through the die plate into the liquid filled chamber (granulator A5 PAC 6, available from GALA GmbH, Germany). Descriptions of the extruder and the granulator may be found in e.g. WO 2004/098305, incorporated herein by reference.

Gum base in the form of pellets and aspartame powder (Aspartame, available from Holland Sweetener Company), in an amount of 1% by weight were added to a hopper at a first inlet of the extruder. Menthol flavor crystals (MENTHOL BPIUSP, available from SHARP MENTHOL INDIA LIMITED, India), in an amount of 3% by weight was dosed to a second inlet and mixed to the gum composition in the extruder. The gum composition had the composition as shown in table 1.

**Table 1.**

| **Ingredient** | **Amount (wt%)** |
|---|---|
| gum base | 96 |
| menthol flavor crystals | 3 |
| aspartame powder | 1 |

The extruder delivered the composition at a feed rate of 400 kg/h to the die plate. The extruder screw speed was 247 rpm. The minimum temperature in the extruder was 44°C and a temperature of less than 70°C was maintained along about ¾ of the extruder barrel length, until the composition passed the heating device in the outlet end of the extruder. Here the composition was heated to an extruder exit temperature of 109°C. The extruder and the granulator produced a pressure difference of 71 bar.

The composition was extruded through the die plate, which was heated to a temperature of 177°C and had 696 holes with a diameter of 0.36 mm. In the granulator chamber the extruded composition was cut to granules by a cutter with 8 blades and cutter speed 1999 rpm. The particles were cooled and transported to the strainer unit (a centrifugal dryer TWS 20, available from GALA GmbH, Germany) in water with temperature 11°C and flow 22 m³/h. The average cooling and transport time in water was approx. 60 seconds. The particle rate was 400 kg/h and the average diameter of the obtained particles was 0.93 mm.

The cooling and transport stage carried out in water in this example could be carried out in other media such as e.g. air as well.

Two examples of actual size distributions of the composition of granules can be seen in table 2. It is seen that the distribution of composition a) is broader than the distribution of composition b).

**Table 2**

| **Sieving diameter (µm)** | **a) Distribution (wt%)** | **b) Distribution (wt%)** |
|---|---|---|
| Above 1400 | 0.31 | 0.01 |
| 1400 - 1250 | 1.09 | 0.26 |
| 1250 - 1000 | 13.15 | 27.86 |
| 1000 - 850 | 65.14 | 71.74 |
| 859 - 710 | 16.77 | 0.11 |
| below 710 | 2.88 | 0.02 |

It should be noted that several parameters such as pressure difference, die plate hole diameter, temperature, cutter speed and more can be varied in this process which will result in resulting granules with a wide variety of properties e.g. the smoothness and the diameter of the granules. Moreover, the size distribution of the composition of granules in table 2 may vary significantly as examples a) and b) in table 2 indicates. A narrow distribution can be preferred; however, distributions as example a) and distributions much broader may still be advantageous according to the invention.

Finally a free-flowing agent is attached to the granules in between de-watering and conveyance to the tablet pressing apparatus or storing or packaging e.g. for transportation.

### Example 3

### Providing the granules with free-flowing agent

The preparation of the chewing gum granules of example 2 was carried out with various free-flowing agents. In one example, the providing of talc as a free-flowing agent was carried out in the following way:

20.0 g of talc was filled into a container. The cut, cooled and expanded granules from the extruder were blown inline into the container and mechanically mixed continuously. At the stage 230.0 g of granules had been added, the container was removed and the content of free-flowing agent was then 8% by weight.

Mainly the same procedure was followed for the other free-flowing agents (the agents are listed in table 3 below) and with other amounts of talc. During the continuous mechanical mixing it was estimated when the amount of granules in the free-flowing agent was suitable for providing a covering layer of free-flowing agent on the surface of all granules in the mix, in most cases corresponding to a saturation of the surface of the granules. The amount of free-flowing agent for each granule was calculated and noted and can be seen in table 3.

For some of the free-flowing agents a large amount of free-flowing agent provided on the surface of each granule was the result, e.g. it is seen that 33% by weight was the result for rice starch, whereas only 1.26% by weight was the result for Mg-stearate.

### Example 4

### Evaluation of different free-flowing agents

The visual evaluation of the flowing properties for the compositions of granules provided with different free-flowing agents in example 3 can be seen in table 3:

**Table 3**

| **Free-flowing agent:** | **Amount (wt%)** | **Flowing properties** |
|---|---|---|
| Rice starch | 33 | Slightly sticking |
| Maizena flour | 8 | OK |
| CMC | 12.7 | Slightly sticking |
| Microcrystalline cellulose | 8 | OK |
| Fine-powdered xylitol | 13.7 | Slightly sticking |
| Tale | 8 | Excellent |
| SiO₂ | 1.35 | Excellent |
| CaCO₃ | 8 | Excellent |
| Mg-stearate | 1.26 | Excellent |

"Excellent" meaning no sticking at all and flowing is completely unhindered by the surrounding granules.

"OK" meaning no sticking at all and flowing is substantially unhindered by the surrounding granules.

"Slightly sticking" meaning that minor sticking between granules can be seen and flowing is slightly hindered but usable.

### Example 5

### Evaluation of different amounts of free-flowing agent

With the composition of granules from example 2 varying amounts of talc was provided on the surface of the granules. The visual evaluation of the flowing properties of these can be seen in table 4:

**Table 4**

| **Amount (wt%)** | **Flowing properties** | **Comments** |
|---|---|---|
| 0.1 | Non-flowing | Highly sticking |
| 0.2 | Non-flowing | Highly sticking |
| 0.5 | Non-flowing | |
| 1 | Non-flowing | |
| 2 | Acceptable | |
| 4 | Excellent | |
| 6 | Excellent | |
| 8 | Excellent | |
| 12 | Excellent | |
| 16 | Excellent | Minor deterioration of texture |
| 20 | Excellent | Deterioration of texture |
| 25 | Excellent | Disintegration of compressed gum |

Talc as free-flowing agent in amounts of less than about 2% by weight showed out to be disadvantageous.

For high amounts of talc the examples showed that the compressed chewing gum comprising chewing gum granules with high amounts of free-flowing agent provided on the surface was more likely to disintegrate upon chewing in only about 15 min.

Similar experiments were carried out for the other free-flowing agents from table 3. For some of the agents, e.g. SiO₂, even 0.5% by weight showed to provide acceptable flowing properties. For none of the free-flowing agents, however, 0.1 or 0.2% by weight provided satisfying free-flowing properties.

### Example 6

### Polyols as free-flowing agent

With the composition of granules from example 2 sorbitol as free-flowing agent was provided on the surface of the granules.

Chewing gum composition wherein the chewing gum granules were provided with 10% by weight of sorbitol as free-flowing agent was tested and the flowing properties were excellent and no sign of disintegration of the compressed chewing gum was seen.

Although sorbitol was used in the example shown here, numerous other polyols may be used with results as satisfying as with sorbitol. This is e.g. the case for xylitol, mannitol, maltitol, isomalt, and erythritol. Moreover, the sugars sucrose, maltose, mannose, glucose and fructose may be used with advantageous results alone or in combination with one or more polyols.

That high amounts of sorbitol or other sweeteners or sugars did not cause the chewing gum to disintegrate is contrary to other non-sweetener free-flowing agents such as talc, SiO₂, and CaCO₃.

### Example 7

### Compressing the compositions into chewing gum tablets

Each of the compositions of chewing gum granules from example 3, 5 and 6 were compressed into a chewing gum according to the following description:

The chewing gum granules were individually mixed in a standard mixer with sweeteners (intense sweeteners: aspartame powder and acesulfame K; bulk sweetener: sorbitol, available from CERESTAR Scandinavia NS, Denmark).

**Table 5.**

| **Mixture for pressed tablets** | |
|---|---|
| **Ingredient** | **Wt%** |
| gum composition granules | 40.58 |
| aspartame powder | 0.14 |
| acesulfame K powder | 0.14 |
| sorbitol powder | 59.14 |

Before pressing, the mixtures passed a standard horizontal vibration sieve removing particles larger than 2.6mm. The mixture was lead to a standard tablet pressing machine comprising dosing apparatus (P 3200 C, available from Fette GmbH, Germany) and pressed into compressed tablets. The tablets were precompressed and then main compressed to a diameter of 16.0mm and a center height of 9mm using a pressing pressure of 33 kN.

The precompression step may in this example and the following examples optionally be omitted in some embodiments.

### Example 8

### Various active ingredients in chewing gum tablets

Single-layer tablets were manufactured similarly to example 7, but now with the formulations indicated in table 6 below. The thickness of the provided tablets was about 8 mm. The composition of the tablets is shown in table 6, wherein the amounts of different active ingredient is maintained for reasons of comparability.

Moreover it should be noted that all tablets BA-BH comprises high-intensity sweeteners in the gum base-containing layer, which according to some embodiments of the invention may be omitted in order to obtain tablets comprising only one active ingredient. Further the mentioned different active ingredients may be combined in both the gum base-containing layer and the gum base-free layer.

**Table 6**

| **Ingredient \ tablet** | **BA** (Wt %) | **BB** (Wt %) | **BC** (Wt %) | **BD** (Wt %) | **BE** (Wt %) | **BF** (Wt %) | **BG** (Wt %) | **BG** (Wt %) | **BH** (Wt %) |
|---|---|---|---|---|---|---|---|---|---|
| Gum composition granules | 38.58 | 38.58 | 38.58 | 38.58 | 38.58 | 38.58 | 38.58 | 38.58 | 38.58 |
| Aspartame powder | 0.14 | 0.14 | 0.14 | 0.14 | 0.14 | 0.14 | 0.14 | 0.14 | 0.14 |
| Acesulfame K powder | 0.14 | 0.14 | 0.14 | 0.14 | 0.14 | 0.14 | 0.14 | 0.14 | 0.14 |
| Sorbitol powder | 59.14 | 59.14 | 59.14 | 59.14 | 59.14 | 59.14 | 59.14 | 59.14 | 59.14 |
| Active ingredient: | | | | | | | | | |
| Cetirizine | 2 | | | | | | | | |
| Nicotine | | 2 | | | | | | | |
| Metformin-HCl | | | 2 | | | | | | |
| Phenylephrine | | | | 2 | | | | | |
| Deca-peptide KSL-W | | | | | 2 | | | | |
| Citric acid | | | | | | 2 | | | |
| Malic acid | | | | | | | 2 | | |
| Vitamin C | | | | | | | | 2 | |
| Resveratrol | | | | | | | | | 2 |

In the tablets BA-BH, Ceterizine and Phenylephrine are applied in the form of hydrochlorides and, where appropriate, conventional buffers were applied.

The examples exhibited advantageous release properties of active ingredients from a single layer tablet thereby availing regulation of taste-masking by means of active ingredients suitable for taste-masking, taste-enhancing by means of active ingredients suitable for taste-enhancing, and general advantageous possibility of controlling the release time of active ingredients whether these are pharmaceuticals or not. Moreover it was noted that the release of the relatively high amount of polyol was advantageous.

### Example 9

### Rheology and sensory evaluation

Each of the compositions of chewing gum tablets from the preceding example 7 were evaluated for their rheology and sensory properties and the result can be seen in table 7.

**Table 7**

| **Free-flowing agent:** | **Amount (Wt%)** | **Rheology and sensory** |
|---|---|---|
| Rice starch | 33 | Less acceptable |
| Maizena flour | 8 | Acceptable |
| CMC | 12.7 | Less acceptable |
| Microcrystalline cellulose | 8 | Acceptable |
| Fine-powdered xylitol | 13.7 | Less acceptable |
| Talc | 4 | Excellent |
| Talc | 8 | Excellent |
| Talc | 12 | Acceptable |
| Talc | 25 | Bad |
| SiO₂ | 1.35 | Excellent |
| CaCO₃ | 8 | Excellent |
| Mg-stearate | 1.26 | Excellent |
| Sorbitol | 20 | Excellent |
| Sorbitol | 50 | Excellent |
| Sorbitol | 75 | Excellent |

"Excellent" means that neither the rheological properties nor the taste of the compressed chewing gum are badly affected at all due to the presence of the free-flowing agent.

"Acceptable" means that the rheological properties of the compressed chewing gum are slightly worsened but the taste of the chewing gum is not affected.

"Less acceptable" means that the rheological properties of the compressed chewing gum are markedly worsened and the taste of the chewing gum is slightly affected. "Bad" means that the rheological properties of the compressed chewing gum are unacceptable.

Different aspect of process parameters, compression materials and release properties are described in the co-pending applications "Multi-modular chewing gum tablet", "High volume compressed chewing gum tablet", "Compressed tablet comprising polyol" and "Compressed chewing gum comprising an encapsulation delivery system comprising natural resin" filed at the same date and hereby incorporated by reference.

## Claims

1. A chewing gum granule containing gum base, said chewing gum granule being substantially ball-shaped, said chewing gum granule having an average diameter below 1900 µm, and the surface of said chewing gum granule being provided with 0.5 to 18% by weight of free-flowing agent.

2. A chewing gum granule according to claim 1, wherein said chewing gum granule has an average diameter above 200 µm.

3. A chewing gum granule according to claim 1 or 2, wherein the surface of said granule is smooth, said granule being substantially without protrusions, depressions or fissures.

4. A chewing gum granule according to any of the claims 1-3, wherein the smooth surface is obtained by means of expansion, e.g. in a liquid media.

5. A chewing gum granule according to any of the claims 1-4, wherein said free-flowing agent has a particle size of less than 80µm, such as between 5 and 40µm.

6. A chewing gum granule according to any of the claims 1-5, wherein said chewing gum granule comprises active ingredients.

7. A chewing gum composition comprising chewing gum granules according to any of the claims 1-6.

8. A chewing gum composition according to claim 7, wherein at least 95% by weight of said chewing gum granules have an average diameter of less than 1900 µm.

9. A chewing gum composition according to claim 7 or 8, wherein said chewing gum granules are substantially homogeneous in shape.

10. A chewing gum composition according to any of the claims 7-9, wherein at least 40%, preferably at least 70%, more preferably at least 95% of said chewing gum granules are substantially ball-shaped.

11. A compressed chewing gum comprising at least one compressed module obtained by compressing a composition according to any of the claims 7-10.

12. A compressed chewing gum according to claim 11, wherein said chewing gum granules are mixed with chewing gum powder obtained through the following steps:
a) cooling of a gum base to a temperature of between about 0 and -273 °C,
b) grinding of the cooled gum base,
c) optional mixing of the powder thus obtained with at least one free-flowing agent, to obtain a chewing gum blend.

13. A method for producing chewing gum granules according to any of the claims 1-6, which method comprises at least the steps of:
a) feeding a gum composition into an extruder;
b) pressurizing the gum composition in the extruder; and
c) extruding the gum composition through a die means.

14. A chewing gum granule according to any of the claims 1-6, wherein said free-flowing agent is selected from the group consisting of talc, CaCO₃, Mg-stearate, SiO₂, gum Arabic, starch such as corn starch, Na-CMC, methylcellulose, saccharide, polyols, fine-powdered bulk sweetener such as xylitol, isomalt or maltitol, active ingredients, any other suitable agent for improving the flowing properties, or any combination thereof.

## Patentansprüche

1. Kaugummi-Körnchen, das Gummibasis enthält, wobei das Kaugummi-Körnchen im Wesentlichen kugelförmig ist, wobei das Kaugummi-Körnchen einen durchschnittlichen Durchmesser unterhalb von 1900 µm aufweist und die Oberfläche des Kaugummi-Körnchens mit 0,5 bis 18 Gew.% fließfähigem Mittel versehen ist.

2. Kaugummi-Körnchen nach Anspruch 1, bei dem das Kaugummi-Körnchen einen durchschnittlichen Durchmesser von mehr als 200 µm aufweist.

3. Kaugummi-Körnchen nach Anspruch 1 oder 2, bei dem die Oberfläche des Körnchens glatt ist, wobei das Körnchen im Wesentlichen ohne Vorsprünge, Mulden oder Risse ist.

4. Kaugummi-Körnchen nach irgendeinem der Ansprüche 1 - 3, bei dem die glatte Oberfläche mittels Expansion, zum Beispiel in einem flüssigen Medium, erhalten wird.

5. Kaugummi-Körnchen nach irgendeinem der Ansprüche 1 -4, bei dem das fließfähige Mittel eine Teilchengröße von weniger als 80 µm, wie zum Beispiel zwischen 5 und 40 µm, aufweist.

6. Kaugummi-Körnchen nach irgendeinem der Ansprüche 1 - 5, bei dem das Kaugummi-Körnchen aktive Bestandteile umfasst.

7. Kaugummi-Zusammensetzung, umfassend Kaugummi-Körnchen nach irgendeinem der Ansprüche 1 - 6.

8. Kaugummi-Zusammensetzung nach Anspruch 7, bei der mindestens 95 Gew.-% der Kaugummi-Körnchen einen durchschnittlichen Durchmesser von weniger als 1900 µm aufweisen.

9. Kaugummi-Zusammensetzung nach Anspruch 7 oder 8, bei der die Kaugummi-Körnchen eine im Wesentlichen homogene Form aufweisen.

10. Kaugummi-Zusammensetzung nach irgendeinem der Ansprüche 7 - 9, bei der mindestens 40 %, vorzugsweise mindestens 70 %, mehr bevorzugt mindestens 95 % der Kaugummi-Körnchen im Wesentlichen kugelförmig sind.

11. Komprimierter Kaugummi, umfassend mindestens ein komprimiertes Modul, das durch Komprimieren einer Zusammensetzung nach irgendeinem der Ansprüche 7 - 10 erhalten ist.

12. Komprimierter Kaugummi nach Anspruch 11, bei dem die Kaugummi-Körnchen mit Kaugummi-Pulver gemischt werden, welches durch die folgenden Schritte erhalten wird:
a) Abkühlen einer Gummibasis auf eine Temperatur zwischen etwa 0 und -273 °C,
b) Mahlen der abgekühlten Gummi-Basis,
c) gegebenenfalls Mischen des so erhaltenen Pulvers mit mindestens einem fließfähigen Mittel, um eine Kaugummi-Mischung zu erhalten.

13. Verfahren zur Herstellung von Kaugummi-Körnchen nach irgendeinem der Ansprüche 1 - 6, wobei das Verfahren mindestens die Schritte umfasst:
a) Einspeisen einer Gummi-Zusammensetzung in einen Extruder;
b) Beaufschlagung der Gummi-Zusammensetzung in dem Extruder mit Druck; und
c) Extrudieren der Gummi-Zusammensetzung durch ein Düsenwerkzeug.

14. Kaugummi-Körnchen nach irgendeinem der Ansprüche 1 - 6, bei dem das fließfähige Mittel ausgewählt ist aus der Gruppe bestehend aus Talkum, CaCO₃, Mg-Stearat, SiO₂, Gummi arabicum, Stärke, wie Maisstärke, Na-CMC, Methylcellulose, Saccharid, Polyolen, feinem pulverförmigem Füllsüßstoff, wie Xylit, Isomalt oder Maltit, aktiven Bestandteilen, irgendeinem anderen geeigneten Mittel zur Verbesserung der Fließeigenschaften oder irgendeiner Kombination derselben.

## Revendications

1. Granulé de gomme à mâcher contenant une base de gomme, ledit granulé de gomme à mâcher étant essentiellement en forme de bille, ledit granulé de gomme à mâcher ayant un diamètre moyen inférieur à 1900 µm, et la surface dudit granulé de gomme à mâcher étant pourvue de 0,5 à 18 % en poids d'agent d'écoulement libre.

2. Granulé de gomme à mâcher selon la revendication 1, dans lequel ledit granulé de gomme à mâcher a un diamètre moyen supérieur à 200 µm.

3. Granulé de gomme à mâcher selon la revendication 1 ou 2, dans lequel la surface dudit granulé est lisse, ledit granulé étant essentiellement sans protubérances, dépressions ni fissures.

4. Granulé de gomme à mâcher selon l'une quelconque des revendications 1 à 3, dans lequel la surface lisse est obtenue par expansion, par exemple, dans un milieu liquide.

5. Granulé de gomme à mâcher selon l'une quelconque des revendications 1 à 4, dans lequel ledit agent d'écoulement libre a une taille de particules inférieure à 80 µm, telle que comprise encre 5 et 40 µm.

6. Granulé de gomme à mâcher selon l'une quelconque des revendications 1 à 5, dans lequel ledit granulé de gomme à mâcher comprend des ingrédients actifs.

7. Composition de gomme à mâcher comprenant des granulés de gomme à mâcher selon l'une quelconque des revendications 1 à 6.

8. Composition de gomme à mâcher selon la revendication 7, dans laquelle au moins 95 % en poids desdits granulés de gomme à mâcher ont un diamètre moyen inférieur à 1900 µm.

9. Composition de gomme à mâcher selon la revendication 7 ou 8, dans laquelle lesdits granulés de gomme à mâcher ont une forme essentiellement homogène.

10. Composition de gomme à mâcher selon l'une quelconque des revendications 7 à 9, dans laquelle au moins 40 %, de préférence au moins 70 %, mieux encore au moins 95 % desdits granulés de gomme à mâcher sont essentiellement en forme de bille.

11. Gomme à mâcher comprimée comprenant au moins un module comprimé obtenu par compression d'une composition selon l'une quelconque des revendications 7 à 10.

12. Gomme à mâcher comprimée selon la revendication 11, dans laquelle lesdits granulés de gomme à mâcher sont mélangés avec une poudre de gomme à mâcher obtenue par les étapes suivantes :
a) le refroidissement d'une base de gomme à une température comprise entre environ 0 et -273°C,
b) le broyage de la base de gomme refroidie,
c) le mélange éventuel de la poudre ainsi obtenue avec au moins un agent d'écoulement libre, pour donner un mélange de gomme à mâcher.

13. Procédé de production de granulés de gomme à mâcher selon l'une quelconque des revendications 1 à 6, procédé qui comprend au moins les étapes consistant à :
a) introduire une composition de gomme dans une extrudeuse ;
b) mettre sous pression la composition de gomme dans l'extrudeuse ; et
c) extruder la composition de gomme à travers une filière.

14. Granulé de gomme à mâcher selon l'une quelconque des revendications 1 à 6, dans lequel ledit agent d'écoulement libre est choisi dans le groupe constitué par le talc, CaCO₃, le stéarate de Mg, SiO₂, la gomme arabique, l'amidon tel que l'amidon de blé, CMC de Na, la méthylcellulose, le saccharide, les polyols, un agent édulcorant en vrac en poudre fine tel que le xylitol, l'isomalt ou le maltitol, des ingrédients actifs, tout autre agent convenant à l'amélioration des propriétés d'écoulement, ou toute combinaison de ceux-ci.
